(19) Europäisches Patentamt
European Patent Office
Office européen des brevets

(11) EP 1 692 297 B1

(12) **EUROPEAN PATENT SPECIFICATION**

(45) Date of publication and mention of the grant of the patent:
**04.01.2012 Bulletin 2012/01**

(21) Application number: **04798537.9**

(22) Date of filing: **17.11.2004**

(51) Int Cl.:
*C12Q 1/00* (2006.01) *C12N 9/06* (2006.01)

(86) International application number:
**PCT/GB2004/004817**

(87) International publication number:
**WO 2005/056815 (23.06.2005 Gazette 2005/25)**

(54) **NITROREDUCTASE BIOSENSORS FOR DETECTING NITRO-COMPOUNDS**

NITROREDUKTASE BIOSENSOREN ZUM NACHWEIS VON NITROVERBINDUNGEN

BIOCAPTEURS A LA NITROREDUCTASE POUR LA DETECTION DE NITRO-COMPOSES

(84) Designated Contracting States:
**AT BE BG CH CY CZ DE DK EE ES FI FR GB GR HU IE IS IT LI LU MC NL PL PT RO SE SI SK TR**

(30) Priority: **11.12.2003 GB 0328784**

(43) Date of publication of application:
**23.08.2006 Bulletin 2006/34**

(73) Proprietors:
- **University Of Wales, Bangor**
  **Wales LL57 2DG (GB)**
- **TRWYN LIMITED**
  **Menai Bridge, Anglesey**
  **Wales LL59 5AH (GB)**

(72) Inventors:
- **KALAJI, Maher**
  **Anglesey,**
  **Wales LL59 5ST (GB)**
- **WILLIAMS, Peter Anthony**
  **Menai Bridge,**
  **Wales LL59 5BU (GB)**
- **GWENIN, Christopher David**
  **Bangor,**
  **Wales LL57 1BB (GB)**

(74) Representative: **Walsh, David Patrick et al**
**Appleyard Lees**
**15 Clare Road**
**Halifax HX1 2HY (GB)**

(56) References cited:
**GB-A- 2 332 432**

- **C.D. GWENIN ET AL.: "The development of a novel amperometric biosensor for the detection of nitro explosives" ABSTRACTS OF THE ANNUAL MEETING OF THE INTERNATIONAL SOCIETY OF BIOCHEMISTRY, [Online] vol. 55, 19 September 2004 (2004-09-19), page 1, XP002314074 Retrieved from the Internet: URL: http://www.google.de/search?q=cache:MNxdOHpOAUgJ:www.isechemistry.gr/showabs.php%3FID%3DS4FP118+kalaji+nitroreductases&hl=de> [retrieved on 2005-01-05]**
- **CHRIS GWENIN: "Poster 50: The Development of an Amperometric Enzyme Sensor for the Detection of Explosives" POSTERS OF THE 2003 YOUNGER EUROPEAN CHEMISTS' CONFERENCE, [Online] 26 August 2003 (2003-08-26), pages 1-19, XP002314075 GRENOBLE 2003 Retrieved from the Internet: URL:http://www.setforeurope.org/grenoble/New%20Folder/posters.htm> [retrieved on 2005-01-05] & E. WHARTON: "The third Younger European Chemists' Conference 26 to 29 August 2003 at the European Synchrotron Radiation Facility (ESRF), Grenoble, France" ANNOUNCEMENT, [Online] 2003, page 1, GRENOBLE Retrieved from the Internet: URL: http://www.cineca.it/hosted/incm/instm /Young_Chemists_Conf_Grenoble_26-290803.ht m> [retrieved on 2005-01-05]**
- **DATABASE Geneseq [Online] 27 February 1996 (1996-02-27), "E. coli nitro reductase gene." XP002314077 retrieved from EBI accession no. GSN:AAQ92891 Database accession no. AAQ92891**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 692 297 B1

- DATABASE EMBL [Online] 2 October 2001 (2001-10-02), "Pseudomonas putida KT2440 flavoprotein (ecd) gene, complete cds." XP002314078 retrieved from EBI accession no. EM_PRO:AF417209 Database accession no. AF417209
- DATABASE EMBL [Online] 21 August 2002 (2002-08-21), "Pseudomonas putida oxygen-insensitive NADPH nitroreductase (pnrA) gene, complete cds." XP002314079 retrieved from EBI accession no. EM_PRO:AF532911 Database accession no. AF532911
- X. REN ET AL.: "A new type of reusable piezoimmunosensor fabricated by a recombinant IgG-binding protein" ANALYST, [Online] vol. 125, 24 February 2000 (2000-02-24), pages 669-671, XP002314076 Retrieved from the Internet: URL: http://pubs.rsc.org/ej/AN/2000/a909736 h.pdf>

## Description

### Field of the Invention

This invention relates to biosensors, methods of sensing nitro-compounds and modified enzymes per se. In particular, but not exclusively the invention relates to biosensors, methods of detecting nitro-compounds and modified enzymes useful in detecting explosives.

### Background to the Invention

Biosensors generally comprise a class of devices that recognise a desired compound (analyte) in a sample and generate a signal which can be resolved to determine the concentration of the compound within the sample. Most biosensors are based on their ability to distinguish a specific analyte, or limited range of analytes, without the need for separation or isolation. For example there are known biosensors which can detect the presence of particular compounds within a blood or water sample directly, thereby eliminating the need for a lengthy or complex purification steps to recover the analyte of interest.

Many biosensors rely on the coupling of a recognition system with an electrochemical or optical transducer to produce an electrical or optical signal or impulse when the recognition system recognises an analyte, which is then used in analyte concentration determination. Electrochemical transducers used in biosensors include potentiometric and amperometric transducer mechanisms. Optical based transducer mechanisms include fluorescence, phosphorescence and a simple colour change.

In potentiometric-based biosensors the accumulation of charge density at the surface of an electrode is measured and is representative of the concentration of analytes to which a biosensor is exposed. In amperometric sensors, electrons that are exchanged between a biological system and an electrode generate a current which may be monitored to determine the concentration of analytes within their sample. Amperometric sensors are commonly employed in blood glucose and ethanol sensors, as well as other devices which monitor compounds of biological significance. In many biosensors a biological recognition molecule performs part of the sensor. The biological recognition molecule may be a nucleic acid sequence, an RNA, or more commonly a protein such as an enzyme or antibody. The biological recognition molecule binds specific analytes, or a limited range of analytes, and is therefore ideally suited for selective detection of specific analytes.

Charge transfer is either accomplished by low molecular weight redox co-factors, such as the $NAD^+$/NADH or by the direct interaction of the redox centres of proteins. Both types of biochemical charge transfer reactions have been previously coupled to redox electrodes. Electrochemistry provides a useful tool for studying the redox chemistry associated with enzymes. A method that can be used to evaluate electron transfer between enzymes and an electrode is the indirect method of using a small redox molecule serving as an electron transfer co-factor. The scheme of the electron transfer coupling is illustrated in figure 1, where the enzymatic ($E_O/E_R$) reaction for the oxidation (or reduction) of the substrate is linked to the electrochemical reduction (or oxidation) of the co-factor ($C_O/C_R$) by the electrode as a final electron acceptor (or donor). The enzyme catalysed electrochemical oxidation (or reduction) of the substrate is called bioelectrocatalysis.

The enzyme electrode is a combination of any electrochemical probe (amperometric, potentiometric or conductometric) with a thin layer (10-200μm) of immobilised enzyme. In these devices, the function of the enzyme is to provide selectivity by virtue of its biological affinity for a particular substrate molecule.

However there are problems in attaching many biological recognition molecules to biosensors. Many biological recognition molecules such as enzymes, and antibodies have "active sites" which must be presented to the analyte in order for the analyte to bind the biological recognition molecule. For many biological recognition molecules, attachment to a biosensor may obscure or hinder the active sites, and therefore render the biological recognition molecule less effective, or, in some cases, inactive.

Of growing interest is the detection of nitro compounds present in many explosives and explosive precursors such as fertiliser.

Interest is growing not only for the detection of buried munitions from previous wars, but also through interest in detecting explosives on a person's body, a person's possessions, vehicles and structures.

For buried or unexploded munitions, there is a great hazard that members of the public working in areas where the explosives are buried, may plough up or step on unexploded ordinance, without being aware that explosives are present at all.

Currently there are sensing systems available to detect explosives, which include for instance the system described in US 5972638, in which a modified organism of the *Pseudomonas* species or *Bacillus* species are sprayed onto ground believed to contain buried explosives. The ground is gently irradiated before spraying on the organism, in order to increase explosive vapour concentration in the soil. This system is relatively expensive, and requires equipment in the form of crop spraying aircraft, spraying devices and irradiation devices. Furthermore, the ground requires substantial quantities of modified organism to be sprayed onto the surface, in order that a detectable signal is achievable.

In WO 97/03201 and GB 2303136, nitroreductase enzymes per se are purified and used to detect nitrates in samples of soil or otherwise, by way of a calorimetric method. The nitroreductase used in these patents is a pentaerythritol tetra nitratereductase, which is specific only for PETN. Furthermore, the concentration level of nitrates which can be detected is relatively high and in order for detection to take place, a soil sample must be removed from the ground, and taken away for a relatively time consuming and complicated assay procedure.

An abstract published with the "Posters of the 2003 Younger European Chemistry Conference, 26 August 2003" in Grenoble mentions "The Development of An Amperometric Enzyme Sensor for the Detection of Explosives".

However, details of the specific enzyme are not given.

Database Geneseq (XP002314077), 27 February 1996 gives the sequence of an "E.coli nitroreductase gene"; database EMBL (XP 002314078), 2 October 2001 gives details of the "Pseudomonas Putida KT2440 flavoprotein (ecd) gene; and Database EMBL (XP002314079), 21 August 2002 gives details of the Pseudomonas Putida oxygen-insensitive NADPH nitroreductase (pnr A) gene. However, no teaching is given of modification of these genes with codons for sulphur containing functional groups or of modification of the encoded nitroreductase enzyme. Similarly, no teaching in relation to their use in a sensing device is given.

Willner et al (Bioelectrochemistry & Bioenergetics, 29 (1992) 29-45) disclose a nitrate biosensor comprising an electrode with immobilised nitroreductase. It would therefore be advantageous to provide an improved biosensor, able to detect explosives in situ, whether in soil or other ground material, or in a sample of material.

It would furthermore be advantageous to provide a highly sensitive detection system for nitro-compounds commonly present in explosives and fertilizers, which is able to detect in at least the nanomolar, but more preferably the picomolar concentration range.

It is an aim of preferred embodiments of the present invention to overcome or mitigate at least one problem of the prior art, whether expressly disclosed herein or not.

## Summary of the Invention

According to a first aspect of the invention there is provided a sensing device as claimed in claim 1.

Preferably the noble metal layer comprises a noble metal selected from the group consisting of gold, silver, platinum, palladium, iridium, rhenium, ruthenium and osmium, or alloys or mixtures thereof. More preferably the noble metal is gold, platinum, or alloys or mixtures thereof, but is most preferably gold.

The noble metal layer preferably comprises at least a top and bottom surface, and suitably the biological material is located on one of the top and bottom surfaces. Preferably the biological material is immobilised on the noble metal layer. The biological material is preferably present as a layer on the noble metal layer. The biological material layer is preferably a self-assembled layer.

Suitably the biological material comprises a plurality of sulphur-containing functional groups, preferably sulphydryl (-SH) groups.

The biological material is an enzyme.

The enzyme is a nitroreductase which has been modified to include a plurality of cysteine residues at a location on the enzyme which does not substantially interfere with the activity of the enzyme.

Suitably the nitroreductases is encoded by the *nfnB* gene (SEQ ID1) in *Escherichia coli* or the *pnrA* gene (SEQ ID2) in *Pseudomonas putida.* (the native enzymes are referred to hereinafter as "*nfnB*" and *"pnrA"* respectively). Suitably the nitroreductase is encoded by a nucleic acid sequence substantially as set out in SEQ ID1 or SEQ ID2.

The biological material may be covered by a fluid permeable cover layer, preferably in the form of membrane. The cover layer may comprise a polycarbonate or polyacrylate material and is preferably between 1 and 100 $\mu$m in thickness, more preferably between 5 and 50 $\mu$m, still more preferably between 8 $\mu$m and 30 $\mu$m and most preferably between 10 and 20$\mu$m.

The noble metal layer is preferably mounted on an insulating substrate. The insulating substrate may be selected from glass, quartz, silicon, insulating polymers, plastics, and mixtures thereof.

The noble metal layer may be connected on a surface not comprising the biological material, to one or more layers of conductive, semi-conductive or insulating material. Conductive and semi-conductive materials include metals, alloys, carbon paste, graphite and conducting polymers such as polpyrrole, polyaniline, polythiophene, polypyrimidine and the like for example. The one or more further layers may be located between the noble metal layer and the insulating substrate, when present.

In particularly preferred examples the sensing device comprises a gold layer on which is self-assembled a layer of the nitroreductase enzyme.

The nitroreductase may comprise substantially the expression product of the nucleic acid sequence shown in SEQ ID3, which comprises the nucleotide sequence between the T7 promoter and the T7 terminator of the pET-28a(+) plasmid containing the *Escherichia coli* K12 *nfnB* gene, in which 6 cysteine residues (a "$Cys_6$" tag) have been inserted at the N-terminal end.

Another preferred nitroreductase comprises substantially the expression product of the nucleic acid sequence shown in SEQ ID5, which is the nucleic acid sequence between the T7 promoter and the T7 terminator on the plasmid pET-28a(+) containing the *Pseudomonas putida* JLR11 *pnrA* gene in which a $Cys_6$ tag has been inserted at the N-terminal end.

The amino acid translation products of SEQ ID3 and SEQ ID5 are given in SEQ ID4 and SEQ ID6 respectively. Suitably the modified nitroreductase comprises a polypeptide sequence substantially as set out in SEQ ID4 or SEQ ID6.

The nitroreductase may be operably associated with an electron mediator, such as a ferrocene, a phthalocyanate or the like, foe example.

According to a second aspect of the invention there is provided a sensing system comprising a sensing device of the first aspect of the invention, mounted in an electrochemical cell.

The electrochemical cell preferably comprises, in addition to the sensing device, a reference electrode. More preferably the electrochemical cell comprises both a reference electrode and a counter-electrode.

The reference electrode may comprise a Calomel electrode (Standard Calomel Electrode (SCE)), $Hg/Hg_2Cl_2$ electrode and/or Ag/AgCl electrode, or any combination thereof. Ag/AgCl electrodes are preferred as they may be manufactured in various forms, for example discs, wires, rods, layers etc.

The electrochemical cell may comprise a housing constructed from glass, polystyrene or the like, for example.

The sensing system may be operably connected to a measuring instrument, such as a voltammeter, amperometer, cyclic voltammeter, or the like, for example.

According to a third aspect of the invention there is provided a method of detecting nitro group-containing compounds, the method comprising the steps of:

(a) providing a sensing device of the first aspect of the invention and a reference electrode;
(b) applying a potential between the electrodes;
(c) measuring the current;
(d) contacting the sensing device with sample of substrate material to be tested; and
(e) measuring the current change.

The method may comprise a further step (f) of subtracting the current change measured with a blank electrode from the value obtained in step (e). The blank electrode may be the sensing device of the first aspect of the invention which either does not contain the biological material, or contains inactivated biological material.

Depending on the physical type of the sensing device of the first aspect of the present invention, there may be a step between steps (a) and (b) of placing the sensing device in a measuring solution. In this case step (d) may comprise adding a sample of the material to be tested, to the measuring solution.

According to a fourth aspect of the present invention there is provided a protein comprising a nitroreductase enzyme which has been modified to comprise a plurality of cysteine residues at a location which does not substantially interfere with its activity incorporated into its structure, which cysteine residues are not present in the native enzyme.

According to a fifth aspect of the present invention there is provided an isolated nucleic acid sequence as claimed in claim 21.

Suitably the nitroreductase gene is selected from the *Escherichia coli* K12 *nfnB* gene and the *Pseudomonas putida* JLR11 *prnA* gene. The nucleic acid sequences for the *nfnB* and *pnrA* genes are given as SEQ ID1 and SEQ ID2 respectively and preferably the nitroreductase gene is encoded by a nucleic acid sequence substantially as set out in SEQ ID1 and SEQ ID2. Suitably the cysteine codons are incorporated at or in the region of the 3' end of the nucleic acid.

According to a sixth aspect of the present invention there is provided a nucleic acid construct as claimed in claim 23.

Suitably the nitroreductase promoter is the T7 promoter from pET-28a(+).

Suitably the construct comprises the pET-28a(+) plasmid.

Preferably the construct is a vector substantially comprising the nucleic acid sequences shown in SEQ ID3 or SEQ ID5, the reverse complement of the said sequences, the complement of the said sequences, the reverse of the said sequences, or sequences having at least 60% sequence identity with the nucleic acid sequences of any one of the aforementioned sequences.

By use of the term "at least 60% identity" it is therefore understood that the invention encompasses more than use of the specific exemplary nucleotide sequences. Modifications to the sequence such as deletions, insertions, or substitutions in the sequence which produce either:

a) "silent" changes which do not substantially affect the functional properties of the protein molecule. For example, alterations in the nucleotide sequence which reflect the degeneracy of the genetic code or which result in the production of a chemically equivalent amino acid at a give site are contemplated, or:
b) promote improvements in activity or modifications in substrate specificity are also contemplated.

A modification of the nucleotide sequence with an identity greater than 80%, preferably more than 85%, more preferably more than 90% and most preferably more than 95% of SEQ ID 1 or 2 is envisaged.

It has been surprisingly found that the incorporation of cysteine resides in nitroreductases enables efficient incorporation of the nitroreductase onto a noble metal electrode which effects sensitivity of nitrocompound detection down to the picomolar concentration range. Most known nitrocompound detection system enable detection down to the nanomolar range only, and it is believed the conjugation of the nitroreductase and noble metal electrode via cysteine linkages enables optimal orientation of the enzyme on the electrode, leading to enhanced sensitivity. The immobilisation of nitroreductase onto noble metal electrodes via introduced cysteine residues, on the enzyme, is also relatively cheap and uncomplicated. The resultant sensing devices are able to be reused many times and can be used *in situ,* or in site to detect buried explosives in ground, or in samples taken from suspected explosive-containing materials.

**EXAMPLES**

For a better understanding of the present invention and to show how embodiments are the same may be put into effect the invention will now be described by way of example only with reference the accompanying drawings in which:

Figure 1 illustrates a general model of a modified electrode, showing the mediation of electron transfer.

Figure 2 illustrates the pET-28a(+) plasmid;

Figure 3 illustrates a partial nucleotide sequence of the pET-28(a)(+) plasmid from the T7 promoter to the T7 terminator region;

Figure 4 illustrates a graph showing the influence of a $Cys_6$ tagged *nfnB* nitroreductase on a buffer solution containing 4-nitrobenzoate and its UV-viz absorbance spectrum;

Figure 5 illustrates a bar chart showing the activity of a $Cys_6$ tagged *nfnB* nitroreductase on various substrates;

Figures 6A and 6B illustrate the activity of desalted and non-desalted forms of a $Cys_6$ modified *nfnB* nitroreductase in catalysing breakdown of 2,4- dinitroethylbenzene;

Figure 7 illustrates activity of a $Cys_6$ modified *nfnB* nitroreductase utilising ferrocene dicarboxylic acid as a cofactor;

Figure 8 illustrates a plot of the activity against increasing concentrations of substrates of a $Cys_6$ modified *nfnB* nitroreductase;

Figure 9 illustrates a cyclic voltammograms of a $Cys_6$ modified *nfnB* nitroreductase immobilised on a golf slide and a control gold slide without attached enzyme;

Figure 10 illustrates cyclic voltammograms of a $Cys_6$ modified *nfnB* nitroreductase utilising ferrocene dicarboxlic acid as a cofactor;

Figure 11 shows the amperometric response of a biosensor utilising *nfnB* $Cys_6$ modified enzyme expressed from a construct at a fixed potential of +100mV; and

Figures 12A and 12B illustrate amperometric measurements of a $Cys_6$ modified *nfnB* nitroreductase containing biosensor with 10, 30, 40 and 50 pmoles 2,4 dinitroethylbenzene.

**Example 1 -** Preparation of a plasmid comprising a modified *nfnB* gene from *Escherichia coli* K12. Plasmids containing *nfnB* genes from *Escherichia coli* K12 modified by addition of codons for a $Cys_6$ tag were prepared in the following manner.

1.1 <u>The procedure for obtaining the original DNA templates</u>

The DNA template was prepared by introducing cells *Escherichia coli* K12 into a solution of TE buffer pH 7.5 (Tris-Cl, ethylenediaminetetraacetic acid (EDTA)) (100 μl, 1%) in an eppendorf tube. The resulting suspension was mixed thoroughly and boiled for 5 min to break down the cell structure, releasing the DNA into the solution, and was then cooled on ice and centrifuged for 2 min. The centrifuge was set to operate at 14000 rpm unless otherwise stated.

1.2 Polymerase chain reaction (PCR) protocol for the nitroreductase DNA

The PCR protocol uses a standard commercial kit (ProofStart™, Qiagen, UK) according to the manufacturer's instructions.

Template DNA (*Escherichia coli* K12) (1 μl) was amplified by PCR using the standard procedure with primers conforming to SEQ ID7 (5 μl), and to SEQ ID8 (5 μl)

The PCR system was programmed to run in the following manner. The system was held at 95°C for 5 min in order to activate the DNA polymerase. The subsequent temperature cycle consisted of 94°C for 30s to separate the DNA strands, 62°C for 1 min for annealing with the primers, 74°C for 2 min for replicating the double stranded DNA. This sequence was repeated for 35 cycles, after which the temperature was held at 74°C enabling any uncompleted double strands to complete. An aliquot of the solution was then run on a 1% agarose gel, in 1x Tris-Borate-EDTA (TBE) buffer pH 8.2 containing ethidium bromide. The ethidium bromide acts as a stain enabling the molecular weight and purity of the DNA to be determined by viewing against a sample containing DNA fragments of known molecular size run in the same gel. The remaining DNA from the PCR was purified to remove the primers, nucleotides, polymerase, and salts, in preparation for other enzymatic reactions as follows.

1.4 Cloning of the nitroreductase gene

Ligation is the incorporation of the DNA into a plasmid. A good efficiency of ligation of foreign DNA into a plasmid can be achieved if both the plasmid and the insert DNA are cut with two different restriction enzymes, which leave single-stranded, cohesive ends. The DNA is thus ligated in only one predetermined direction.

The PCR product (5 μl) was mixed with the appropriate restriction enzymes (2 μl of each), purified water (2 μl), and 10x appropriate restriction buffer solution (1 μl). A separate solution was made up of the expression plasmid pET-28a(+) (5 μl) (Novagen, UK; Figures 2 and 3). The region between the T7 promoter and T7 terminator is shown in Figure 3. This was mixed with the same two restriction enzymes (1 μl of each) plus a third, *Eco*RI (1 μl), purified water (1 μl), and 10x buffer solution (1 μl): the *Eco*RI digestion ensures that the pET28a(+) plasmid does not religate without an insert. Each solution was incubated at 37° for 1 hour to allow the restriction digestion to occur, after which each was cleaned up according to section 1.3. Both were dried under vacuum and redissolved in purified water (4 μl).

The two solutions were then mixed and DNA ligase (1 μl), and ligase 10x buffer solution (1 μl), were added. This solution was then maintained overnight at 16°C for the ligation process. To check that the ligation of the PCR product between the T7 promoter and T7 terminator of pET-28(a)(+) was successful an aliquot of the ligation mix was digested with the appropriate restriction enzymes (1 μl each) as described above and subjected to agarose gel electrophoresis.

The remaining ligation mixture was mixed with cells of competent *E. coli* DH5α (200 μl), an efficient strain of *E. coli* for plasmid maintenance. In order to transform the recombinant plasmid into the competent cells, the mixture was left on ice for 30 min, was then heated to 42°C for exactly 50 sec and then returned to ice for 2 min. The resulting culture was added to Luria-Bertani (LB) medium (500 μl), incubated at 37°C for 45 min, and then applied to the Petri dishes.

1.5 Growing the colonies

The solid medium consisted of LB agar containing the antibiotic kanamycin (50 μg/ml). This medium will only allow bacteria carrying the recombinant plasmid to grow, as a kanamycin resistance gene is an integral part of pET28a (+). The transformed *E. coli* culture was spread onto the plates in a range of different concentrations. Single colonies, which grew were then transferred to liquid LB medium (5 ml) containing kanamycin (50 μg/ml) and grown overnight at 37°C.

The recombinant plasmid was isolated from the overnight cultures after separating the cells as a pellet following centrifugation. A standard QIAprep Spin Miniprep Kit (Qiagen, UK) was used to purify the plasmid according to the manufacturer's instructions.

1.7 PCR protocol for the incorporation of the $Cys_6$ sequences.

Using purified pCDG1 as template DNA, the protocol for PCR was repeated using designed primers conforming to SEQ ID9 and to SEQ ID8: these contain six adjacent codons for Cys at the 3' end of the nitroreductase such that when the gene is expressed an amino acid sequence of $Cys_6$ is added to the N-terminus of the protein, between the

$His_6$-tag determined by pET28a(+) and the start codon of the nitroreductase. The same protocols as described above for cloning the modified gene, transforming it into *E. coli* DH5α, and purifying it were used. The resultant plasmid was named pMKS2.

**Example 2** - Expression of the $Cys_6$- modified nitroreductase enzyme prepared in section 1.7, coded by the modified nfnB gene located in plasmid pET-28a(+) prepared in Example 1.

2.1 Expression of the enzymes

Using the protocol of Section 1.4, the plasmids prepared in Example 1 (2 μl) were transformed into competent cells of the Rossetta strain of *E. coli* (200 μl), which is an efficient bacterium for the expression of heterologous genes.

The bacteria containing the plasmids were grown overnight at 37°C in 500 ml of LB plus added kanamycin (50 μg/ml), until an optical density ($O.D._{600nm}$) of 0.6 was achieved. Expression of the cloned genes was induced by addition of isopropyl-beta-D-thiogalactopyranoside (IPTG) (2 ml, 0.1 M: 0.4 $mM^{(final)}$), and grown for a further four hours at 37°C.

The cells were then harvested by centrifuging (8000 rpm for 10 min) and the resulting pellets were placed on ice and resuspended in imidazole solution (10 mM, 10 ml) consisting of phosphate buffer (pH 7.4, 6.25 ml, 0.1 M), and imidazole (2 M, 0.25 ml), made up to 50 ml with distilled water. The resulting suspensions were then sonicated four times for 30s, to break open the cells, whilst avoiding overheating the solution. The solutions were then centrifuged (35000 rpm, 5°C, for 45 min). The resulting solution contains the nitroreductase (NTR) and the pellet contains the cell debris. The solutions were then run on a sodium dodecyl sulphate-polyacrylamide gel electrophoresis to check that the protein was overproduced and that its molecular weight was as expected.

2.2 The enzyme purification protocol

The engineered proteins carry a $His_6$ tag at their N-termini, making it easier to purify the protein by eluting the solution through a nickel-agarose column, where the histidine residues bind to $Ni^{2+}$ embedded in the resin. His-tagged target proteins are thus selectively retained on the column of nickel agarose, and can be eluted (competitively removed) with imidazole, which competes for Ni binding sites, displacing the protein.

For elution from the column, imidazole solutions (8 ml) were prepared with increasing concentrations from 50 mM to 1.0 M in sterile filtered distilled water.

The extract of the cells was added to the column dissolved in phosphate buffer containing 10 mM imidazole. Elution was carried out according to the maker's instructions (Amersham Biosciences U.K.) using imidazole concentrations increasing in stepped amounts. Each eluate was collected in 1 ml samples (5 for each concentration) to avoid dilution. Finally, the column was washed with the remaining binding buffer (4 ml) and stored below 5°C ready for reuse. The second ml of each elute was run on a SDS-PAGE gel along with the sample flow-through and an induced unpurified sample.

2.3 The removal of imidazole from the protein

The imidazole from the elution stages remains in the enzyme solution. It was removed, as stated below, and the nitroreductase (NTR) resuspended in Tris buffer pH 7.2 ready for use on the electrode surface. A PD-10 desalting column (Amersham Biosciences U.K.) which is a gravity-operated polypropylene column containing 8.5 ml of Sephadex™ G-25 Medium, with a bed height of 5 cm, used for desalting and buffer exchange and was used according to the manufacturer's instructions.

**Example 3 -** Characterisation of Enzyme Activity. The activity of the modified nitroreductase enzyme prepared in Example 1 was preferred as follows:

3.1 Nitroreductase assay

A UV assay and associated spectra were carried out on the NTR obtained at the end of Section 2.3 in solution (≅1.0 $\mu M^{(Final)}$, 10 μl), in a cuvette with buffer tris-HCl pH 7.4 (50 mM, 500 μl), NADPH (1 mM, 100 μl), nitroaromatic compound (substrate, various conc.), and flavin mononucleotide (FMN) (1 mM, 5 μl), made up to 1 ml with distilled water at 25 °C.

The spectra were collected at a scan rate of 500 nm/min between 220-500 nm resulting in 1 min scans, and the assays were run at 340 nm for 2 min each, using an Uvikon 943 double beam spectrophotometer. The parameters of the spectra and assays are as noted above unless otherwise stated. All spectral measurements were carried out against a blank consisting of the assay solution detailed above, but lacking the nitroreductase enzyme, and all assay measurements were carried out against a blank consisting of the assay solution lacking the substrate, unless otherwise stated.

**Example 4 -** Preparation of an enzyme biosensor utilising the modified nitroreductase of Example 2.

4.1 Preparation of the gold sheet for UV-vis

Gold was used to prove the concept that the modified enzymes can be immobilised via the thiol groups to gold substrate and remain active after the immobilisation. Gold sheets were cleaned in a 50:50 mixture of concentrated sulphuric and nitric acid overnight. Desalted and non-desalted Cys-tagged NTR enzymes were each/separately adsorbed onto a gold slide, 3 mm by 5 mm. The gold sheet was left for 24 hours in the nitroreductase solution, and then immersed in buffer (pH 7) to remove any residual proteins.

4.2 Electrochemical procedure

Electrochemical measurements were performed using an Autolab PGstat3. The analysis was carried out with a three-electrode cell, using a Saturated Calomel reference electrode (SCE) and a platinum mesh counter electrode. All glassware was cleaned using a 50:50 mixture of concentrated $H_2SO_4$: $HNO_3$ followed by rinsing in purified water, cleaning in a steam bath, and drying in the oven. The working electrode was a gold slide with a self-assembled layer of the appropriate enzyme.

The cell contained sodium phosphate buffer pH 7.1 (20 ml, 0.1 M), mixed with the co-factor dicarboxylic ferrocene (50 □M). Additions of the substrate were made by pipetting the desired quantities and concentrations in through the top of the cell.

4.3 Preparation of gold-coated glass slides for electrochemical measurements

The gold-coated glass slides obtained from Gold Arrandee™ / Au(111) uses the borosilicate glass (AF45) base material, which is 1.1 +/- 0.1 mm in thickness with the size of the glass slide being 11 x 11 +/- 0.2 mm. The special glass substrate is well suited for the flame annealing procedure which is used to obtain Au(111) terraces. A thin (2.5 +/- 1.5 nm) adhesive layer of chromium is applied to the glass surface. This layer guarantees optimum adhesion of the gold layer to the glass. On top of this thin Chromium layer a final gold layer is applied which is 250 +/- 50 nm thick.

Prior to use, the gold-coated slides were flame-annealed in a Bunsen burner until they attained red heat several times. After cooling in air for a short period of time, the slide was quenched in ultrapure water. The slides were then dipped into an enzyme-containing solution for 24 hrs at 5°C to assemble the layer of the enzyme. Each slide was then washed in tris-buffer prior to transfer to an electrochemical cell.

5.1 Assay results

The influence of the nitroreductase on a buffer solution containing 4-nitrobenzoate (625 μM) was assessed using UV-vis; the nitroreductase (10 μl) was placed in the cuvette prior to run 2. The results are shown in Figure 4.

The scans show a reduction in the intensity of absorbance at 340 nm on the second scan, followed by reduction of the absorbance in subsequent scans, down to -0.29 absorbance units (a.u.). The peak at 300 nm (4-nitrobenzoate) increased in intensity on the second scan due to introduction and corresponding absorbance of nitroreductase, then decreased on subsequent scans. The use of increasing amounts of nitroreductase afforded a corresponding increase in NADPH conversion, indicating that the nitroreductase is responsible for the oxidation of NADPH.

As substrates, 4-nitrotoluene, 2,4-dinitrotoluene, 2-ethylhexyl nitrate and nitrobenzene were good substrates whereas 4-nitrobenzoate, 1,2-dinitrobenzene and 2,4-dinitroethylbenzene were excellent substrates (Figure 5).

Both desalted and non-desalted forms of the protein NfnB-cys1 nitroreductase with cysteine tags as prepared hereinabove were adsorbed onto gold slides and scans of the activity towards 2,4-dinitroethylbenzene (DNEB) (620 μmoles) were assessed. The results are shown in Figures 6A and 6B which clearly illustrate the effective catalysis by the desalted enzyme (Fig. 6A).

NADPH is not an efficient co-factor in electrochemical cells because the oxidised form $NADP^+$ produced in the enzyme-catalysed reaction cannot be stoichiometrically reduced to a biologically active form of NADPH electrochemically. For this reason ferrocene dicarboxylic acid (1μm) was used to eliminate this problem, as it exhibits good electrochemical reversibility. The results are shown in Figure 7, using 300μm DNEB with nitroreductase.

The oxidation of the dicarboxylic ferrocene can be seen in the reduced absorbance with time at 280 nm indicating that the ferrocene derivative is being oxidised via the enzymatic reaction, hence, can be utilised as the nitroreductase co-factor.

5.2 Specific activity of nitroreductase

The specific activity of nitroreductase was assessed by calculating the rates at different concentrations of substrate in association with the different nitroreductase concentration. The preferred substrate used for both the His-tagged nitroreductase from E. coli (NfnB-his1) and the further modified Cys-tagged enzyme (NfnB-cys1) was 2,4-dinitroethylbenzene (31 μM). The protein concentration was calculated by placing a micro protein-PR™ reagent (1 ml) in 3 cuvettes, a blank, a standard (500 mg/l), and a sample of nitroreductase. UV measurements were taken at 600 nm in accordance with the procedure. The unpurifed protein was compared against the purified protein. Four runs were performed for each volume of 5, 10, 15, and 20 μl of protein.

The activity for the enzyme NfnB-his1 before purification was 0.22 □moles/min/mg which rose to 4.65 □moles/min/mg following purification. Hence, the purification achieved an approximate average of 20-fold increase in activity, with the enzyme making up only 7.6% of the unpurifed solution. The relevant specific activities are tabulated in Table 1.

Table 1: The concentration and specific activity for the unpurified and purified proteins NfnB-his1 and NfnB-cys1 with 2,4-dinitroethylbenzene.

| Protein | Average total protein (□g/ml) | Average specific activity (□moles/min/mg) |
|---|---|---|
| **NfnB-his1** | | |
| **Unpurifed** | 5.23 | 0.22 |
| **Purified** | 0.40 | 4.64 |
| **NfnB-cys1** | | |
| **Unpurifed** | 4.83 | 0.18 |
| **Purified** | 0.18 | 2.92 |

The protein with the $Cys_6$ tags (NfnB-cys1) showed that the insertion of the Cys residues reduced the activity by an average of ≈37%. After the removal of imidazole from the protein solutions containing the Cys tags, the proteins retained approximately 86% of its original activity.

5.3 $K_m$ and $V_{max}$ of the nitroreductase

In order to calculate the $K_m$ and $V_{max}$ values, a plot was constructed of activity in □□˜min/mg against increasing concentration of substrates as shown in Figure 8.

The resulting data was used to calculate the $K_m$ and $V_{max}$ values from Direct Linear method via an enzyme kinetics theory and practice software package (Enzpack). The resulting data was averaged and obtained with a 68% confidence level, as shown in Table 2 below.

Table 2 Resulting $K_M$ and $V_{max}$ values for the *E coli* nitroreductase

| Proteins | $K_m$ (μmol) | $V_{max}$ (μmol/min/mg) | $V_{max}/K_m$ ratio | Substrate |
|---|---|---|---|---|
| **NfnB-his1** | 27 | 27 | 0.99 | 2,4-dinitroethylbenzene |
| **NfnB-cys1** | 33 | 27 | 0.84 | 2,4-dinitroethylbenzene |

The values of $K_m$ and $V_{max}$ are specific to each enzyme however, the ratio $V_{max}/K_m$ can be used to compare the enzyme efficiency, as shown in Table 2. The efficiency of the enzyme is ultimately limited by the rate of diffusion of the substrate to the enzyme and by the chemical events that occur in the active site of the enzyme.

The number of moles of substrate converted to product per unit of time, known as the turnover number ($K_{cat}$), also allows comparisons between the enzymes. The turnover number can be calculated from the following equation:

$$V_{max} = K_{cat}[E_{Tot}]$$

The average $K_{cat}$ of substrate for the two modified NfnB was 6.4 x $10^2$ mol/min/moles of enzyme.

5.4 Evaluation of the biosensor; voltammetry results

Cyclic voltammograms were obtained for the unmodified gold electrode and the gold electrode modified with the desalted enzyme solution; a seal was made between the working electrode and the electrolyte solution with *o*-rings defining a geometric area of 0.6 $cm^2$. The modification of the electrodes was achieved by immersing the gold slide, which is now the working electrode, in the enzyme solution (0.1 M phosphate butter, pH 7.1) for 24 hours, then thoroughly rinsing with sodium phosphate buffer. The results are shown in Figure 9.

The reduction in the oxidation peak (oxidation of gold surface) at +350 mV is caused by the formation of the layer of enzymes blocking the electrode surface. The presence of the layer is also manifested by the large reduction in the current associated with the hydrogen evolution reaction at -400 mV. In order for the reaction (reduction of nitroaromatics) to proceed, a co-factor needs to be introduced into the solution. A voltammogram of the co-factor, dicarboxylic ferrocene (35μm) in 0.1 M phosphate butter, pH 7.1, was carried out to evaluate an appropriate potential to hold the amperometric sensor at, so that the dicarboxylic ferrocene could be reduced after it has been oxidised during the enzymatic reaction. The results are shown in Figure 10.

Figure 11 shows the amperometric response of the biosensor with the NfnB-cys1 enzyme expressed from pMKS2 which was evaluated at a fixed potential of +100 mV in a 0.1 M phosphate buffer (pH 7.1) and the co-factor was dicarboxylic ferrocene (10 mM, 200 □moles). The working electrode was a gold slide modified with NfnB-cys1. and all potential values are quoted against a SCE. Prior to the injection of DNEB (20 μl of 2 □M solution of DNEB resulting in a concentration of 2 nM in the cell), the current was allowed to stabilise for 730 s until a steady state current was reached. After the addition of the DNEB sample, a slight increase in current was observed, thought to be due to convection caused by introducing the substrate.

After the initial rise, the current becomes less positive by approximately 10nA. This drop was caused by the reduction of the ferrocenium dicarboxylic acid that had been formed as a result of the oxidation of ferrocene dicarboxylic acid by the oxidised form of the nitroreductase. The current then starts to decay to a value close to the initial baseline value thus allowing successive readings to be taken. Further amperometric measurements were carried out with 10, 30, 40, and 50 pmoles of DNEB corresponding to 0.5 nM, 1.5 nM, 2 nM, 2.5 nM respectively. The larger the amount of DNEB that is introduced into the system, the larger is the drop. A linear relationship is found between the magnitude of the current and the concentration of the analyte; thus providing a basis for an amperometric sensor. The results are shown in Figures 12A and 12B. No response was obtained when DNEB was not present in the analyte solution.

Figure 12A shows amperometric data for the four different concentrations of DNEB at a potential of +100 mV in a 0.1 M phosphate buffer (pH 7.1), the working electrode was a gold slide modified with NfnB-cys1. DNEB samples were injected 100 s after applying the potential; the co-factor was ferrocene dicarboxylic (10 mM, 200 μmoles). Figure 12B shows a plot of the magnitude of the current drop taken 70 s after the injection of DNEB from the data in Figure 12A against the concentration of DNEB. The plot is that of a straight line and shows a linear relationship between the magnitude of the current and the concentration of the analyte.

The lowest concentration examined in this case corresponds to a concentration of DNEB in the parts per trillion range (ppt).

**Example 6**

The above protocols and tests from Sections 1.1 to 5.4 were performed utilising the *Pseudomonas putida* JLR11 *prnA* gene conforming to SEQ ID2 in a pET-28a(+) expression vector comprising the expression sequence shown in SEQ ID4. The results again indicated that excellent sensitivity, in the picomolar range, was exhibited by the resultant biosensor.

**Conclusions**

The results illustrate that the introduction of the cysteine tags at the N-terminus does not reduce the activity in a way that (detrimentally affects/prevents) amperometric measurements, and that the tags were successful in the immobilisation of the enzyme to a gold surface, without the loss of activity. Evidence for the assembly of the nitroreductases on the gold surface was obtained by FTIR, UV-vis spectroscopy, and cyclic voltammetry.

The nitroreductase was shown to be active with a range of nitroaromatics and a nitro ester, namely 2-ethylhexyl nitrate, and afforded different rates of reaction for each substrate. The optimum pH (pH 7.1) and temperature (<40 °C) for the enzyme were established along with $K_M$, $V_{max}$, and turnover numbers ($K_{cat}$).

The response of the amperometric sensor was in the nanoamp range and detection was unexpectedly down in the parts per trillion region. The drop in current (and the rate of drop) was found to be proportional to the concentration of nitroaromatics in solution, and the system showed evidence of recovery after each sample, allowing successive samples to be taken. In addition, the enzyme remained active when kept in the fridge for a period of two weeks.

## Claims

1. A sensing device comprising an electrode comprising a noble metal layer, on which layer is located a nitroreductase enzyme, wherein the enzyme comprises a plurality of introduced cysteine residues at a location which does not substantially interfere with its activity.

2. A sensing device as claimed in Claim 1 wherein the noble metal layer comprises a noble metal selected from the group consisting of gold, silver, platinum, palladium, iridium, rhenium, ruthenium and osmium, or alloys or mixtures thereof.

3. A sensing device as claimed in Claim 1 or 2 wherein the enzyme is immobilised on the noble metal layer.

4. A sensing device as claimed in any one of Claims 1 to 3 wherein the enzyme is present as a layer on the noble metal layer.

5. A sensing device as claimed in Claim 1 wherein the nitroreductase is encoded by a nucleic acid sequence with an identity greater than 80% of SEQ ID1 or SEQ ID2.

6. A sensing device as claimed in any preceding claim wherein the enzyme is covered by a fluid permeable cover layer.

7. A sensing device as claimed in Claim 6 wherein the cover layer comprises a polycarbonate or polyacrylate material.

**8.** A sensing device as claimed in any preceding claim wherein the noble metal layer is mounted on an insulating substrate.

**9.** A sensing device as claimed in any preceding claim wherein the noble metal layer is connected on a surface not comprising the enzyme, to one or more layers of conductive, semi-conductive or insulating material.

**10.** A sensing device as claimed in any preceding claim comprising a gold layer on which is self-assembled a layer of nitroreductase enzyme which has been modified to include a plurality of cysteine residues at a location on the enzyme which does not substantially interfere with the activity of the enzyme.

**11.** A sensing device as claimed in Claim 10 wherein the nitroreductase enzyme comprises substantially the expression product of the nucleic acid sequence shown in SEQ ID3 or SEQ ID5.

**12.** A sensing device as claimed in Claim 10 wherein the nitroreductase enzyme comprises a polypeptide sequence as set out in SEQ ID4 or SEQ ID6.

**13.** A sensing device as claimed in any one of Claims 10 to 12 wherein the nitroreductase is operably associated with an electron mediator.

**14.** A sensing system comprising a sensing device of any one of Claims 1 to 13, mounted in an electrochemical cell.

**15.** A sensing system as claimed in Claim 14 wherein the electrochemical cell comprises, in addition to the sensing device, a reference electrode.

**16.** A sensing system as claimed in Claim 15 wherein the electrochemical cell further comprises a counter-electrode.

**17.** A method of detecting nitro group containing compounds, the method comprising the steps of:

(a) providing a sensing device of any one of Claims 1 to 15 and a reference electrode;
(b) applying a potential between the electrodes;
(c) measuring the current;
(d) contacting the sensing device with a sample of substrate material to be tested; and
(e) measuring the current change.

**18.** A method as claimed in Claim 17 further comprising a step (f) of substracting the current change measured with a blank electrode from the value obtained in step (e).

**19.** A method as claimed in Claim 17 or 18 further comprising a step between steps (a) and (b)of placing the sensing device in a measuring solution.

**20.** A protein comprising a nitroreductase enzyme which has been modified to comprise a plurality of cysteine residues at a location which does not substantially interfere with its activity incorporated into its structure, which cysteine residues are not present in the native enzyme.

**21.** An isolated nucleic acid sequence comprising a nitroreductase gene modified by the addition of a plurality of codons for cysteine residues incorporated in the nucleotide sequence encoding the nitroreductase in such a way that the cysteine residues do not substantially interfere with the activity of the enzyme.

**22.** A nitroreductase enzyme as claimed in Claim 20 encoded by a nucleic acid sequence with an identity greater than 80% of SEQ ID1 or SEQ ID2 modified by the addition of a plurality of codons for cysteine residues.

**23.** A nucleic acid construct comprising:

(a) a promoter for the expression of a nitroreductase gene;
(b) a nucleotide sequence of a nitroreductase gene having a plurality of codons for cysteine residues incorporated in the nucleotide sequence encoding the nitroreductase in such a way that the cysteine residues do not substantially interfere with the activity of the enzyme.

**24.** A nucleic acid construct as claimed in Claim 23 comprising the nucleic acid sequence set out in SEQ ID3 or SEQ ID5, the reverse complement of said sequences, the complement of said sequences, the reverse of said sequences or sequences having at least 60% sequence identity with the nucleic acid sequences of any one of said sequences.

## Patentansprüche

**1.** Sensoreinheit, umfassend eine Elektrode, umfassend eine Edelmetallschicht, auf welcher ein Nitroreduktase-Enzym angeordnet ist, wobei das Enzym eine Vielzahl von eingeführten Cysteinresten an einem Ort, der seine Aktivität nicht wesentlich stört, umfasst.

**2.** Sensoreinheit gemäß Anspruch 1, wobei die Edelmetallschicht ein Edelmetall umfasst, ausgewählt aus der Gruppe, bestehend aus Gold, Silber, Platin, Palladium, Iridium, Rhenium, Ruthenium und Osmium, oder Legierungen oder Gemische davon.

**3.** Sensoreinheit gemäß Anspruch 1 oder 2, wobei das Enzym auf der Edelmetallschicht immobilisiert ist.

**4.** Sensoreinheit gemäß einem der Ansprüche 1 bis 3, wobei das Enzym als Schicht auf der Edelmetallschicht vorliegt.

**5.** Sensoreinheit gemäß Anspruch 1, wobei die Nitroreduktase von einer Nukleinsäuresequenz mit einer Identität von mehr als 80 % zu SEQ ID1 oder SEQ ID2 codiert wird.

**6.** Sensoreinheit gemäß einem der vorstehenden Ansprüche, wobei das Enzym von einer fluiddurchlässigen Deckschicht bedeckt ist.

**7.** Sensoreinheit gemäß Anspruch 6, wobei die Deckschicht ein Polycarbonat- oder Polyacrylatmaterial umfasst.

**8.** Sensoreinheit gemäß einem der vorstehenden Ansprüche, wobei die Edelmetallschicht auf einem isolierenden Substrat befestigt ist.

**9.** Sensoreinheit gemäß einem der vorstehenden Ansprüche, wobei die Edelmetallschicht an einer Oberfläche, die das Enzym nicht umfasst, mit einer oder mehreren Schichten von leitendem, halbleitendem oder isolierendem Material verbunden ist.

**10.** Sensoreinheit gemäß einem der vorstehenden Ansprüche, umfassend eine Goldschicht, auf der eine Schicht von Nitroreduktase-Enzym, das modifiziert wurde, um eine Vielzahl von Cysteinresten an einem Ort des Enzyms, der die Aktivität des Enzyms nicht wesentlich stört, zu enthalten, selbstorganisiert ist.

**11.** Sensoreinheit gemäß Anspruch 10, wobei das Nitroreduktase-Enzym im Wesentlichen das Expressionsprodukt der in SEQ ID3 oder SEQ ID5 gezeigten Nukleinsäuresequenz umfasst.

**12.** Sensoreinheit gemäß Anspruch 10, wobei das Nitroreduktase-Enzym eine Polypeptidsequenz gemäß SEQ ID4 oder SEQ ID6 umfasst.

**13.** Sensoreinheit gemäß einem der Ansprüche 10 bis 12, wobei die Nitroreduktase mit einem Elektronenvermittler operativ verbunden ist.

**14.** Sensorsystem, umfassend eine Sensoreinheit gemäß einem der Ansprüche 1 bis 13, die in einer elektrochemischen Zelle befestigt ist.

**15.** Sensorsystem gemäß Anspruch 14, wobei die elektrochemische Zelle zusätzlich zu der Sensoreinheit eine Referenzelektrode umfasst.

**16.** Sensorsystem gemäß Anspruch 15, wobei die elektrochemische Zelle ferner eine Gegenelektrode umfasst.

**17.** Verfahren zum Nachweisen von Nitrogruppenenthaltenden Verbindungen, wobei das Verfahren die Schritte umfasst:

(a) Bereitstellen einer Sensoreinheit gemäß einem der Ansprüche 1 bis 15 und einer Referenzelektrode;

(b) Anlegen eines Potentials zwischen den Elektroden;
(c) Messen des Stroms;
(d) Kontaktieren der Sensoreinheit mit einer Probe von zu prüfendem Substratmaterial; und
(e) Messen der Stromänderung.

**18.** Verfahren gemäß Anspruch 17, ferner umfassend einen Schritt (f) des Subtrahierens der mit einer Leerelektrode gemessenen Stromänderung von dem in Schritt (e) erhaltenen Wert.

**19.** Verfahren gemäß Anspruch 17 oder 18, ferner umfassend zwischen den Schritten (a) und (b) einen Schritt des Anordnens der Sensoreinheit in einer Messlösung.

**20.** Protein, umfassend ein Nitroreduktase-Enzym, das modifiziert wurde, um eine Vielzahl von Cysteinresten an einem Ort, der seine Aktivität nicht wesentlich stört, in seiner Struktur einverleibt zu umfassen, wobei die Cysteinreste in dem nativen Enzym nicht vorhanden sind.

**21.** Isolierte Nukleinsäuresequenz, umfassend ein Nitroreduktase-Gen, modifiziert durch das derartige Hinzufügen einer Vielzahl von Codons für Cysteinreste in die Nucleotidsequenz, die die Nitroreduktase codiert, dass die Cysteinreste die Aktivität des Enzyms nicht wesentlich stören.

**22.** Nitroreduktase-Enzym gemäß Anspruch 20, codiert von einer Nukleinsäuresequenz mit einer Identität von mehr als 80 % zu SEQ ID1 oder SEQ ID2, modifiziert durch das Hinzufügen einer Vielzahl von Codons für Cysteinreste.

**23.** Nukleinsäurekonstrukt, umfassend:

(a) einen Promoter für die Expression eines Nitroreduktase-Gens;
(b) eine Nucleotidsequenz eines Nitroreduktase-Gens mit einer Vielzahl von Codons für Cysteinreste, die der Nucleotidsequenz, die die Nitroreduktase codiert, derartig einverleibt sind, dass die Cysteinreste die Aktivität des Enzyms nicht wesentlich stören.

**24.** Nukleinsäurekonstrukt gemäß Anspruch 23, umfassend die Nukleinsäuresequenz gemäß SEQ ID3 oder SEQ ID5, das reverse Komplement der Sequenzen, das Komplement der Sequenzen, das Reverse der Sequenzen oder Sequenzen mit wenigstens 60 % Sequenzidentität mit der Nukleinsäuresequenz einer dieser Sequenzen.

## Revendications

**1.** Dispositif capteur comprenant une électrode comprenant une couche de métal noble, couche sur laquelle est située une enzyme nitroréductase, l'enzyme comprenant une pluralité de résidus de cystéine introduits sur une position qui n'interfère pas sensiblement avec son activité.

**2.** Dispositif capteur tel que revendiqué dans la revendication 1, dans lequel la couche de métal noble comprend un métal noble choisi dans le groupe consistant en l'or, l'argent, le platine, le palladium, l'iridium, le rhénium, le ruthénium et l'osmium, ou les alliages ou mélanges de ceux-ci.

**3.** Dispositif capteur tel que revendiqué dans la revendication 1 ou 2, dans lequel l'enzyme est immobilisée sur la couche de métal noble.

**4.** Dispositif capteur tel que revendiqué dans l'une quelconque des revendications 1 à 3, dans lequel l'enzyme est présente sous forme d'une couche sur la couche de métal noble.

**5.** Dispositif capteur tel que revendiqué dans la revendication 1, dans lequel la nitroréductase est codée par une séquence d'acide nucléique ayant une identité supérieure à 80 % avec SEQ ID1 ou SEQ ID2.

**6.** Dispositif capteur tel que revendiqué dans l'une quelconque des revendications précédentes, dans lequel l'enzyme est recouverte d'une couche de couverture perméable aux fluides.

**7.** Dispositif capteur tel que revendiqué dans la revendication 6, dans lequel la couche de couverture comprend un matériau de type polycarbonate ou polyacrylate.

8. Dispositif capteur tel que revendiqué dans l'une quelconque des revendications précédentes, dans lequel la couche de métal noble est montée sur un substrat isolant.

9. Dispositif capteur tel que revendiqué dans l'une quelconque des revendications précédentes, dans lequel la couche de métal noble est connectée, sur une surface ne comprenant pas l'enzyme, à une ou plusieurs couches d'un matériau conducteur, semi-conducteur ou isolant.

10. Dispositif capteur tel que revendiqué dans l'une quelconque des revendications précédentes, comprenant une couche d'or sur laquelle est auto-assemblée une couche de l'enzyme nitroréductase qui a été modifiée de façon à comprendre une pluralité de résidus de cystéine sur une position, sur l'enzyme, qui n'interfère pas sensiblement avec l'activité de l'enzyme.

11. Dispositif capteur tel que revendiqué dans la revendication 10, dans lequel l'enzyme nitroréductase comprend sensiblement le produit d'expression de la séquence d'acide nucléique présentée dans SEQ ID3 ou SEQ ID5.

12. Dispositif capteur tel que revendiqué dans la revendication 10, dans lequel l'enzyme nitroréductase comprend une séquence polypeptidique telle que présentée dans SEQ ID4 ou SEQ ID6.

13. Dispositif capteur tel que revendiqué dans l'une quelconque des revendications 10 à 12, dans lequel la nitroréductase est associée d'une manière opérationnelle à un médiateur d'électrons.

14. Système capteur comprenant un dispositif capteur de l'une quelconque des revendications 1 à 13, monté dans une cellule électrochimique.

15. Système capteur tel que revendiqué dans la revendication 14, dans lequel la cellule électrochimique comprend, en plus du dispositif capteur, une électrode de référence.

16. Système capteur tel que revendiqué dans la revendication 15, dans lequel la cellule électrochimique comprend en outre une contre-électrode.

17. Procédé de détection de composés contenant un groupe nitro, le procédé comprenant les étapes de :

(a) mise à disposition d'un dispositif capteur de l'une quelconque des revendications 1 à 15 et d'une électrode de référence ;
(b) application d'un potentiel entre les électrodes ;
(c) mesure de l'intensité du courant ;
(d) mise en contact du dispositif capteur avec un échantillon du matériau substrat à tester ; et
(e) mesure du changement d'intensité du courant.

18. Procédé tel que revendiqué dans la revendication 17, comprenant en outre une étape (f) de soustraction, de la valeur obtenue dans l'étape (e), du changement d'intensité du courant mesuré avec une électrode à blanc.

19. Procédé tel que revendiqué dans la revendication 17 ou 18, comprenant en outre entre les étapes (a) et (b) une étape de mise en place du dispositif capteur dans une solution de mesure.

20. Protéine comprenant une enzyme nitroréductase qui a été modifiée de façon à comprendre une pluralité de résidus de cystéine sur une position qui n'interfère pas sensiblement avec son activité, incorporés dans sa structure, les résidus de cystéine n'étant pas présents dans l'enzyme native.

21. Séquence d'acide nucléique isolée comprenant un gène de la nitroréductase modifié par addition d'une pluralité de codons pour des résidus de cystéine incorporés dans la séquence nucléotidique codant pour la nitroréductase, de façon que les résidus de cystéine n'interfèrent pas sensiblement avec l'activité de l'enzyme.

22. Enzyme nitroréductase telle que revendiquée dans la revendication 20, codée par une séquence d'acide nucléique ayant une identité supérieure à 80 % avec SEQ ID1 ou SEQ ID2, modifiée par addition d'une pluralité de codons pour des résidus de cystéine.

23. Construction d'acide nucléique comprenant .

(a) un promoteur pour l'expression d'un gène de la nitroréductase ;
(b) une séquence nucléotidique d'un gène de la nitroréductase, ayant une pluralité de codons pour des résidus de cystéine incorporés dans la séquence nucléotidique codant pour la nitroréductase, de telle sorte que les résidus de cystéine n'interfèrent pas sensiblement avec l'activité de l'enzyme.

**24.** Construction d'acide nucléique telle que revendiquée dans la revendication 23, comprenant la séquence d'acide nucléique présentée dans SEQ ID3 ou SEQ ID5, le complément inverse desdites séquences, le complément desdites séquences, l'inverse desdites séquences, ou des séquences ayant une identité de séquence d'au moins 60 % avec les séquences d'acides nucléiques de l'une quelconque desdites séquences.

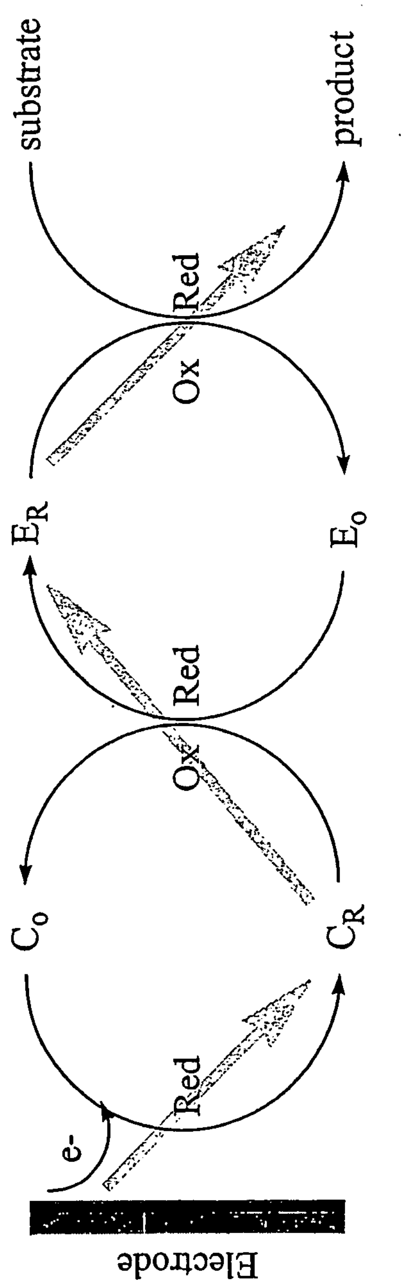


Figure 1

Figure 2

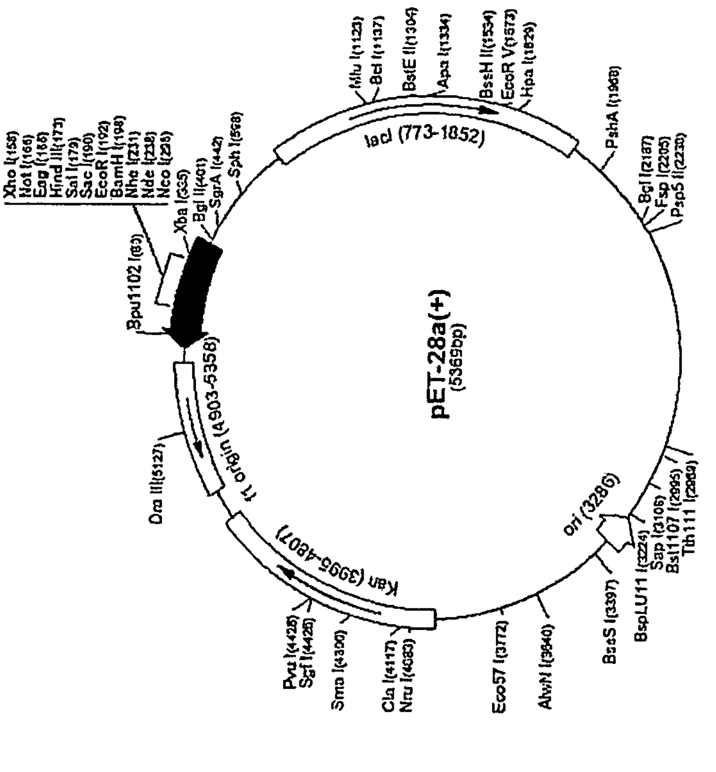
pET-28a(+)
(5369bp)
lacI (773-1852)
Kan (3995-4807)
f1 origin (4903-5358)
ori (3286)
Xho I(158)
Not I(166)
Eag I(166)
Hind III(173)
Sal I(179)
Sac I(190)
EcoR I(192)
BamH I(198)
Nhe I(231)
Nde I(238)
Nco I(296)
Xba I(335)
Bgl II(401)
SgrA I(442)
Sph I(598)
Mlu I(1123)
Bcl I(1137)
BstE II(1304)
Apa I(1334)
BssH II(1534)
EcoR V(1573)
Hpa I(1629)
PshA I(1968)
Bgl I(2187)
Fsp I(2205)
Psp5 II(2230)
Tth111 I(2969)
Bst1107 I(2995)
Sap I(3106)
BspLU11 I(3224)
BssS I(3397)
AlwN I(3640)
Eco57 I(3772)
Nru I(4083)
Cla I(4117)
Sma I(4300)
Sgf I(4426)
Pvu I(4426)
Dra III(5127)
Bpu1102 I(80)

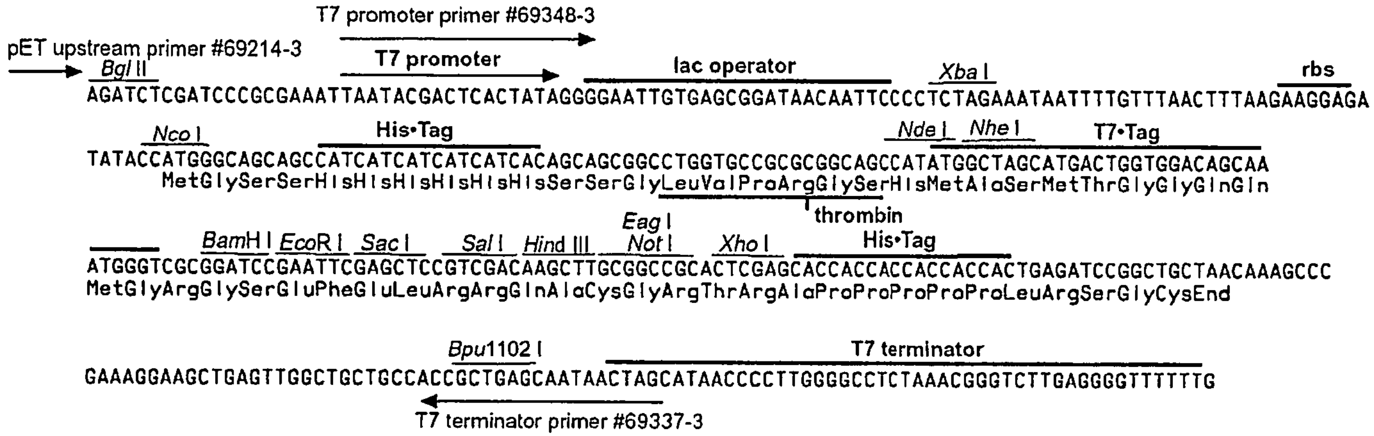
T7 promoter primer #69348-3
pET upstream primer #69214-3
Bgl II
T7 promoter
lac operator
Xba I
rbs
AGATCTCGATCCCGCGAAATTAATACGACTCACTATAGGGGAATTGTGAGCGGATAACAATTCCCCTCTAGAAATAATTTTGTTTAACTTTAAGAAGGAGA
Nco I
His•Tag
Nde I
Nhe I
T7•Tag
TATACCATGGGCAGCAGCCATCATCATCATCATCACAGCAGCGGCCTGGTGCCGCGCGGCAGCCATATGGCTAGCATGACTGGTGGACAGCAA
MetGlySerSerHisHisHisHisHisHisSerSerGlyLeuValProArgGlySerHisMetAlaSerMetThrGlyGlyGlnGln
thrombin
Eag I
BamH I
EcoR I
Sac I
Sal I
Hind III
Not I
Xho I
His•Tag
ATGGGTCGCGGATCCGAATTCGAGCTCCGTCGACAAGCTTGCGGCCGCACTCGAGCACCACCACCACCACCACTGAGATCCGGCTGCTAACAAAGCCC
MetGlyArgGlySerGluPheGluLeuArgArgGlnAlaCysGlyArgThrArgAlaProProProProProLeuArgSerGlyCysEnd
Bpu1102 I
T7 terminator
GAAAGGAAGCTGAGTTGGCTGCTGCCACCGCTGAGCAATAACTAGCATAACCCCTTGGGGCCTCTAAACGGGTCTTGAGGGGTTTTTTG
T7 terminator primer #69337-3

Figure 3

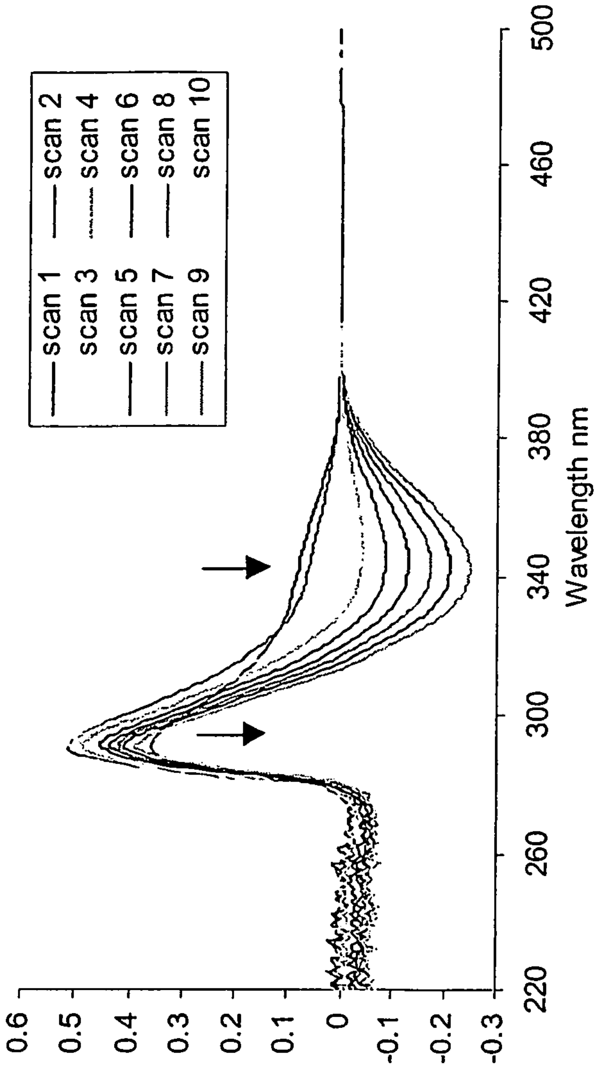
scan 1
scan 2
scan 3
scan 4
scan 5
scan 6
scan 7
scan 8
scan 9
scan 10
Absorbance
0.6
0.5
0.4
0.3
0.2
0.1
0
-0.1
-0.2
-0.3
220
260
300
340
380
420
460
500
Wavelength nm

**Figure 4**

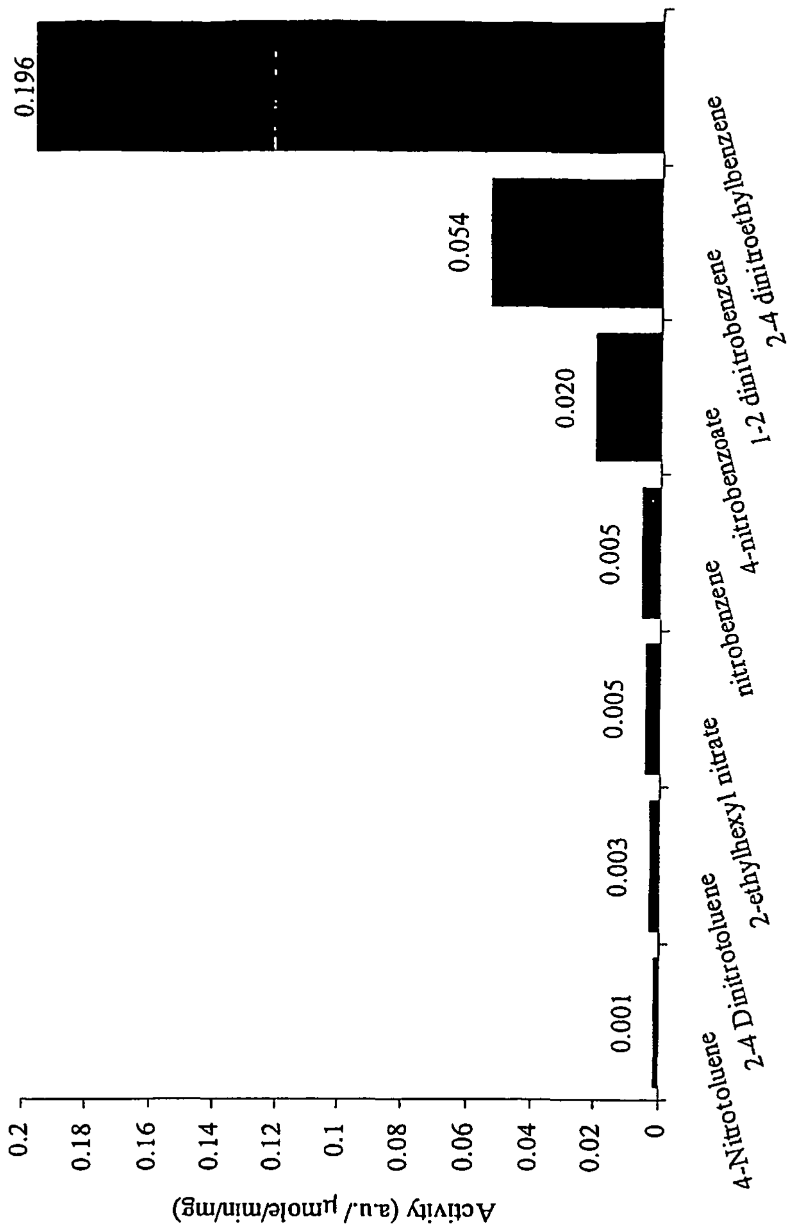
Activity (a.u./ µmole/min/mg)
0.2
0.18
0.16
0.14
0.12
0.1
0.08
0.06
0.04
0.02
0
0.001
0.003
0.005
0.005
0.020
0.054
0.196
4-Nitrotoluene
2-4 Dinitrotoluene
2-ethylhexyl nitrate
nitrobenzene
4-nitrobenzoate
1-2 dinitrobenzene
2-4 dinitroethylbenzene

Figure 5

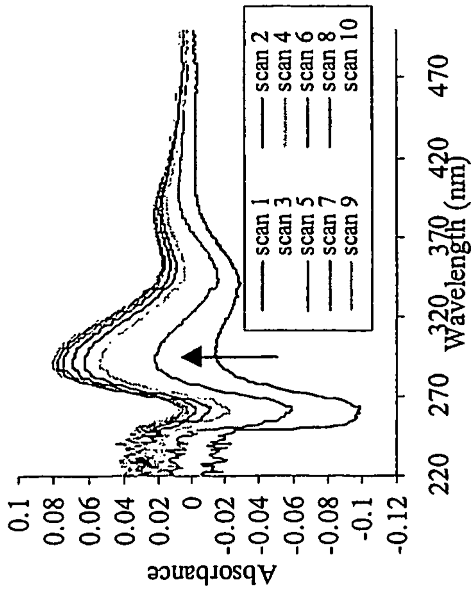


Figure 6A

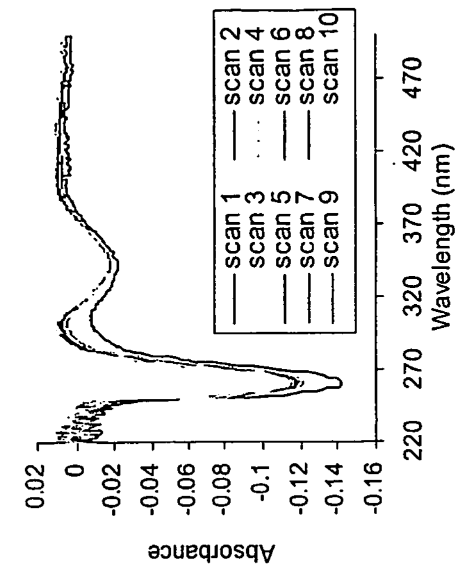


Figure 6B

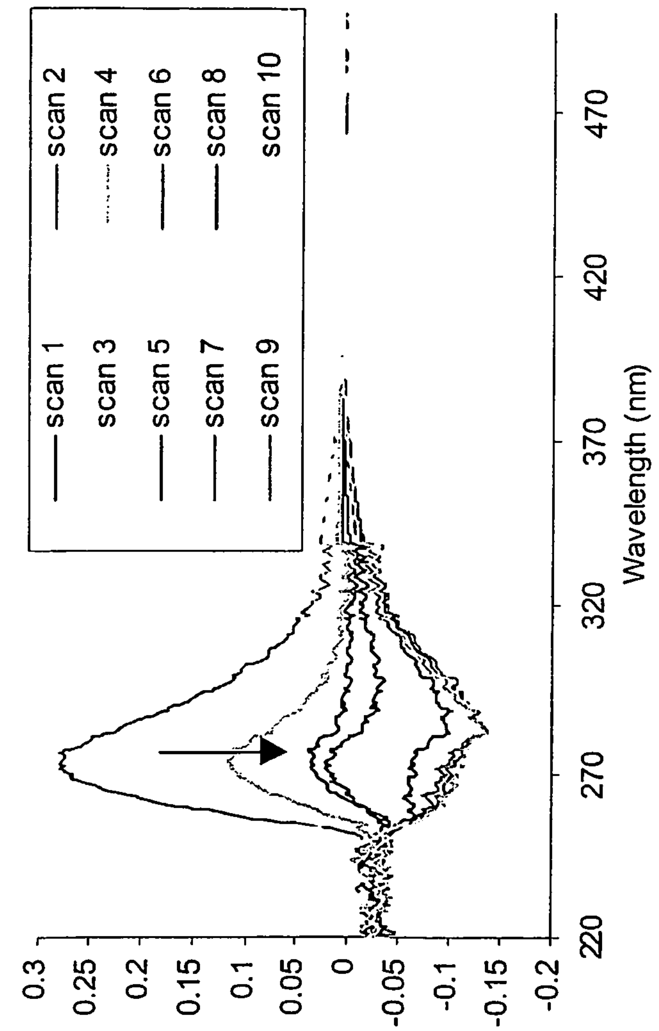
Absorbance
0.3
0.25
0.2
0.15
0.1
0.05
0
-0.05
-0.1
-0.15
-0.2
220
270
320
370
420
470
Wavelength (nm)
scan 1
scan 2
scan 3
scan 4
scan 5
scan 6
scan 7
scan 8
scan 9
scan 10

**Figure 7**

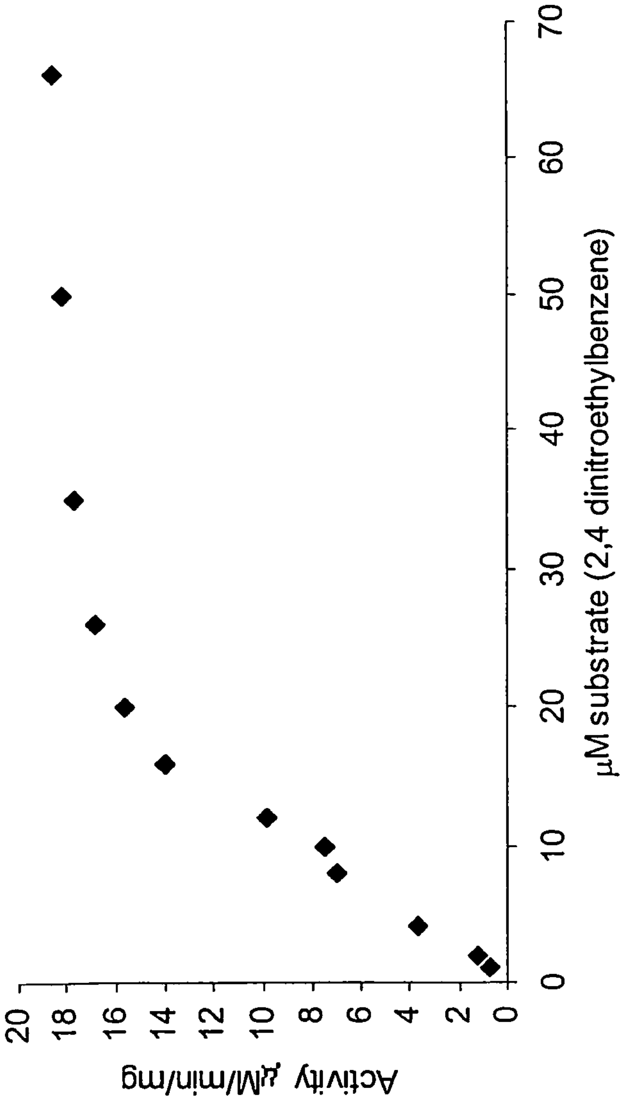
Activity µM/min/mg
20
18
16
14
12
10
8
6
4
2
0
0
10
20
30
40
50
60
70
µM substrate (2,4 dinitroethylbenzene)

**Figure 8**

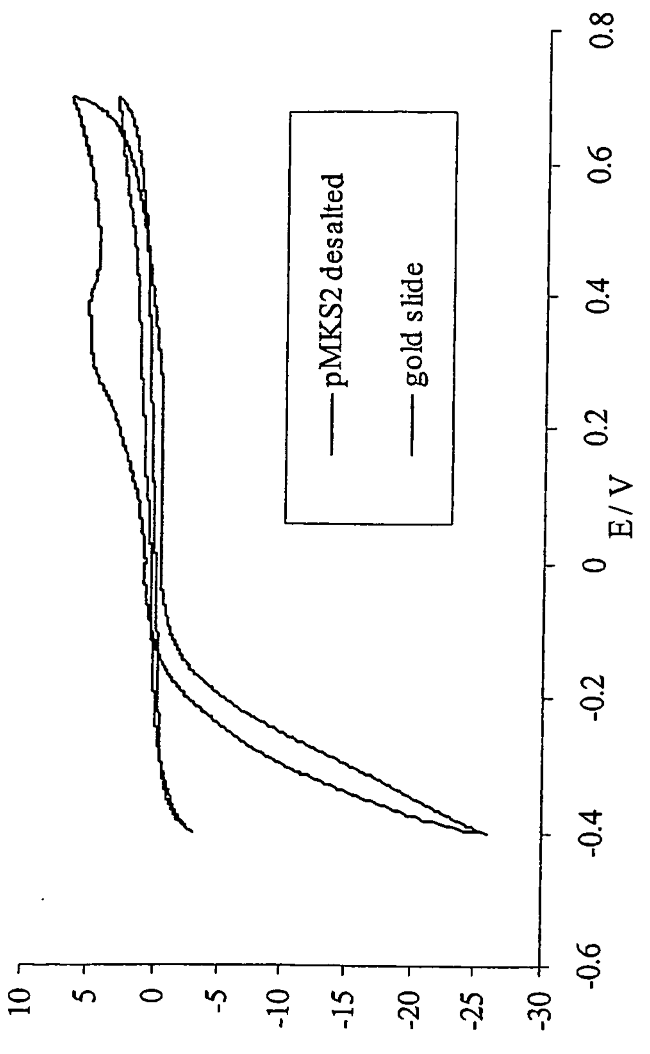
i / mA
10
5
0
-5
-10
-15
-20
-25
-30
E / V
-0.6
-0.4
-0.2
0
0.2
0.4
0.6
0.8
pMKS2 desalted
gold slide

Figure 9

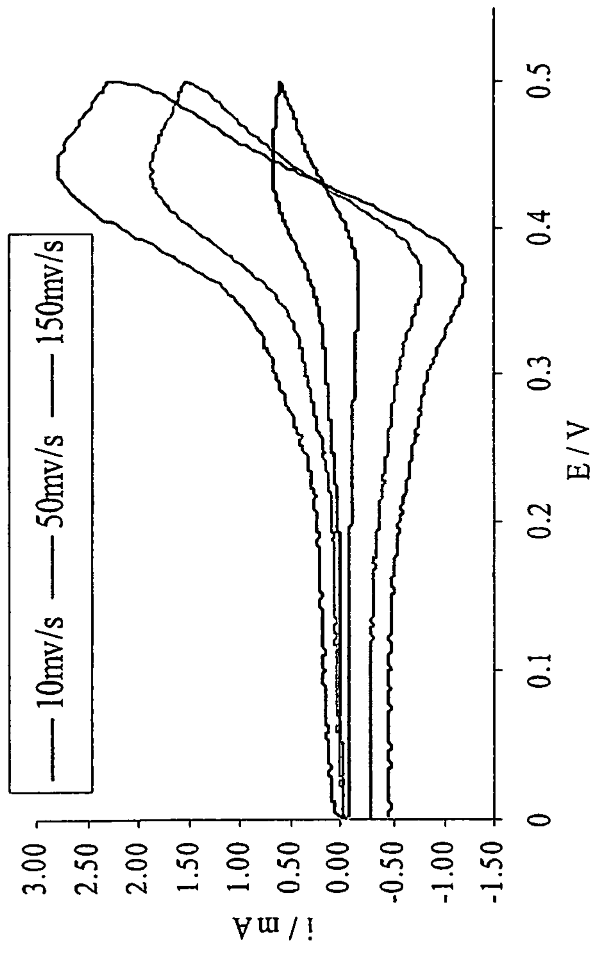
10mv/s
50mv/s
150mv/s
i / mA
3.00
2.50
2.00
1.50
1.00
0.50
0.00
-0.50
-1.00
-1.50
0
0.1
0.2
0.3
0.4
0.5
E / V

Figure 10

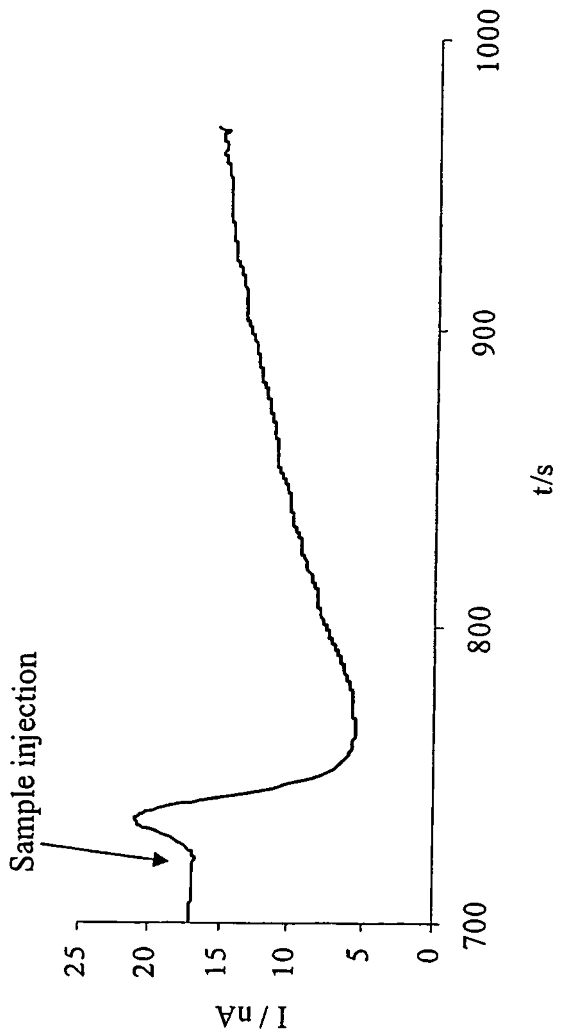


Figure 11

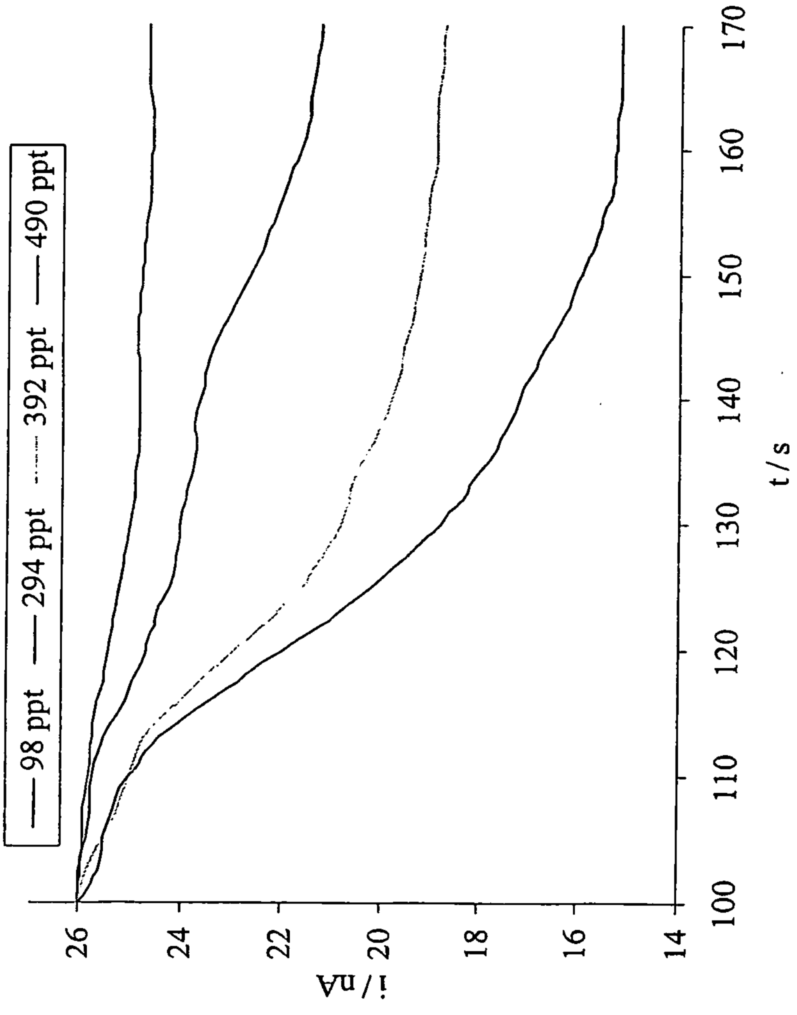
98 ppt
294 ppt
392 ppt
490 ppt
i / nA
26
24
22
20
18
16
14
100
110
120
130
140
150
160
170
t / s

Figure 12A

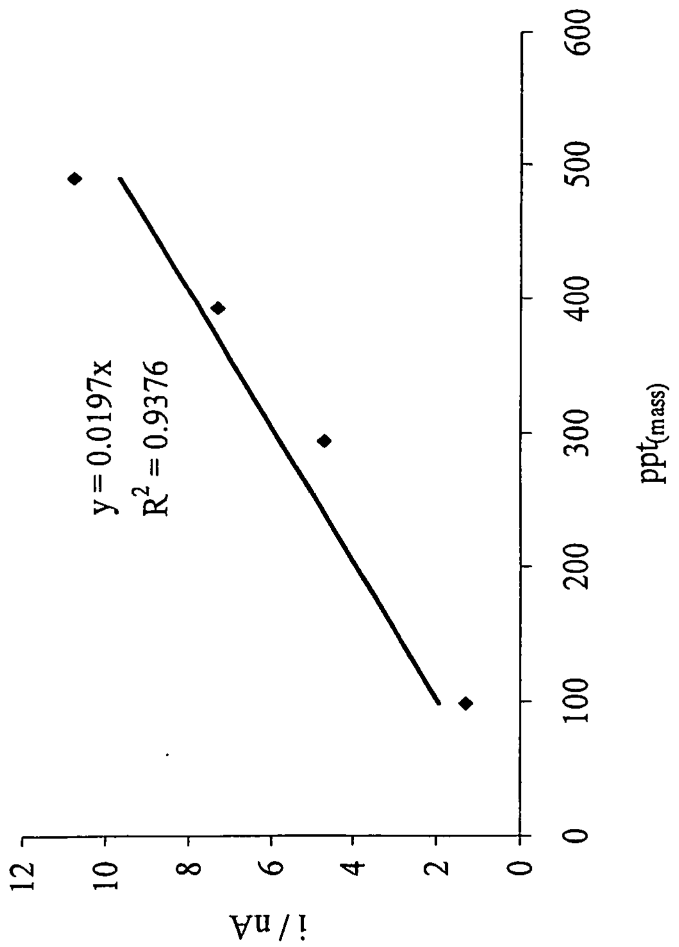
y = 0.0197x
$R^2$ = 0.9376
i / nA
0
2
4
6
8
10
12
0
100
200
300
400
500
600
$ppt_{(mass)}$

Figure 12B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5972638 A
- WO 9703201 A
- GB 2303136 A


### Non-patent literature cited in the description


- Posters of the 2003 Younger European Chemistry Conference. *The Development of An Amperometric Enzyme Sensor for the Detection of Explosives,* 26 August 2003
- **Willner et al.** *Bioelectrochemistry & Bioenergetics,* 1992, vol. 29, 29-45

SEQUENCE LISTING

<110> University of Wales, Bangor
Trwyn Ltd

<120> Improvements In and Relating to Biosensors

<130> BA/SLH/Y1861

<160> 9

<170> PatentIn version 3.1

<210> 1
<211> 654
<212> DNA
<213> Escherichia coli K12

<400> 1

```
atggatatca tttctgtcgc cttaaagcgt cattccacta aggcatttga tgccagcaaa      60

aaacttaccc cggaacaggc cgagcagatc aaaacgctac tgcaatacag cccatccagc     120

accaactccc agccgtggca ttttattgtt gccagcacgg aagaaggtaa agcgcgtgtt     180

gccaaatccg ctgccggtaa ttacgtgttc aacgagcgta aaatgcttga tgcctcgcac     240

gtcgtggtgt tctgtgcaaa aaccgcgatg gacgatgtct ggctgaagct ggttgttgac     300

caggaagatg ccgatggccg ctttgccacg ccggaagcga aagccgcgaa cgataaaggt     360

cgcaagttct tcgctgatat gcaccgtaaa gatctgcatg atgatgcaga gtggatggca     420

aaacaggttt atctcaacgt cggtaacttc ctgctcggcg tggcggctct gggtctggac     480

gcggtaccca tcgaaggttt tgacgccgcc atcctcgatg cagaatttgg tctgaaagag     540

aaaggctaca ccagtctggt ggttgttccg gtaggtcatc acagcgttga agattttaac     600

gctacgctgc cgaaatctcg tctgccgcaa aacatcacct taaccgaagt gtaa           654
```

<210> 2
<211> 826
<212> DNA
<213> Pseudomanas putida JLR11

<400> 2

```
atgagccttc aagacgaagc actcaaagcc tggcaagccc gttatggcga gccagctaac      60
ttacctgctg ccgacaccgt gatcgcgcag atgttgcagc atcgatcagt acgtgcctac     120
agcgatcttc ctgtggatga gcagatgctg agctgggcga tcgcggcggc ccagtcagcc     180
tcgacttcct cgaacctgca agcttggagc gtgctcgccg tgcgggatcg cgagcgtctc     240
gcgaggcttg cccgactgtc cggtaaccag cgccatgtcg agcaggcacc gctgttcctg     300
gtctggctcg tggactggtc acgcctacgc cgactagcca gaacccttca ggcaccgact     360
gcaggtatcg actatttaga aagctacacc gtcggtgttg tagatgcagc tctggccgct     420
cagaacgccg cactagcttt cgaggcccaa ggactgggaa tcgtttacat cggcggaatg     480
cgcaaccacc cggaagcgat gtccgaggag cttggcctgc caaacgacac tttcgctgta     540
tttggcatgt gcgtcggtca tcccgatccg gcacagcccg ccgagatcaa gccacgcctg     600
gcgcaatcag tggtgcttca ccgtgagcgc tatgaggcca ccgaggcaga ggcggtttca     660
gttgctgcct atgaccgaag gatgagcgac ttccaacatc gtcaacaacg cgaaaaccgt     720
tcctggtcca gccaggccgt ggaacgtgta aaaggagcgg attcactgag cggaagacac     780
cgcttgcgag atgcattaaa caccctaggt ttcggcctgc gctgag                   826
```

<210> 3
<211> 1066
<212> DNA
<213> Escherichia coli K12 nfnB in pET-28(a)(+); pMKS2

<220>
<221> CDS
<222> (88)..(858)
<223> Coding sequence for nfnB gene

<220>
<221> misc_feature
<222> (250)..(267)
<223> Cys tags

<220>
<221> misc_feature <222> (160)..(177)
<223> His tags

<220>
<221> misc_feature
<222> (268)..(285)
<223> primer

<220>
<221> misc_feature
<222> (996)..(1010)
<223> primer

<400> 3

```
taatacgact cactataggg gaattgtgag cggataacaa ttcccctcta gaaataattt     60

tgtttaactt taagaaggag atatacc atg ggc agc agc cat cat cat cat cat    114
                              Met Gly Ser Ser His His His His His
                              1               5

cac agc agc ggc ctg gtg ccg cgc ggc agc cat atg gct agc atg act      162
His Ser Ser Gly Leu Val Pro Arg Gly Ser His Met Ala Ser Met Thr
10                  15                  20                  25

ggt gga cag caa atg ggt cgc gga tcc tgt tgc tgt tgc tgt tgc gat      210
Gly Gly Gln Gln Met Gly Arg Gly Ser Cys Cys Cys Cys Cys Cys Asp
                30                  35                  40

atc att tct gtc gcc tta aag cgt cat tcc act aag gca ttt gat gcc      258
Ile Ile Ser Val Ala Leu Lys Arg His Ser Thr Lys Ala Phe Asp Ala
            45                  50                  55

agc aaa aaa ctt acc ccg gaa cag gcc gag cag atc aaa acg cta ctg      306
Ser Lys Lys Leu Thr Pro Glu Gln Ala Glu Gln Ile Lys Thr Leu Leu
        60                  65                  70
```

```
caa tac agc cca tcc agc acc aac tcc cag ccg tgg cat ttt att gtt      354
Gln Tyr Ser Pro Ser Ser Thr Asn Ser Gln Pro Trp His Phe Ile Val
    75                  80                  85

gcc agc acg gaa gaa ggt aaa gcg cgt gtt gcc aaa tcc gct gcc ggt      402
Ala Ser Thr Glu Glu Gly Lys Ala Arg Val Ala Lys Ser Ala Ala Gly
90                  95                  100                 105

aat tac gtg ttc aac gag cgt aaa atg ctt gat gcc tcg cac gtc gtg      450
Asn Tyr Val Phe Asn Glu Arg Lys Met Leu Asp Ala Ser His Val Val
                110                 115                 120

gtg ttc tgt gca aaa acc gcg atg gac gat gtc tgg ctg aag ctg gtt      498
Val Phe Cys Ala Lys Thr Ala Met Asp Asp Val Trp Leu Lys Leu Val
            125                 130                 135

gtt gac cag gaa gat gcc gat ggc cgc ttt gcc acg ccg gaa gcg aaa      546
Val Asp Gln Glu Asp Ala Asp Gly Arg Phe Ala Thr Pro Glu Ala Lys
        140                 145                 150

gcc gcg aac gat aaa ggt cgc aag ttc ttc gct gat atg cac cgt aaa      594
Ala Ala Asn Asp Lys Gly Arg Lys Phe Phe Ala Asp Met His Arg Lys
    155                 160                 165

gat ctg cat gat gat gca gag tgg atg gca aaa cag gtt tat ctc aac      642
Asp Leu His Asp Asp Ala Glu Trp Met Ala Lys Gln Val Tyr Leu Asn
170                 175                 180                 185

gtc ggt aac ttc ctg ctc ggc gtg gcg gct ctg ggt ctg gac gcg gta      690
Val Gly Asn Phe Leu Leu Gly Val Ala Ala Leu Gly Leu Asp Ala Val
                190                 195                 200

ccc atc gaa ggt ttt gac gcc gcc atc ctc gat gca gaa ttt ggt ctg      738
Pro Ile Glu Gly Phe Asp Ala Ala Ile Leu Asp Ala Glu Phe Gly Leu
            205                 210                 215

aaa gag aaa ggc tac acc agt ctg gtg gtt gtt ccg gta ggt cat cac      786
Lys Glu Lys Gly Tyr Thr Ser Leu Val Val Val Pro Val Gly His His
        220                 225                 230

agc gtt gaa gat ttt aac gct acg ctg ccg aaa tct cgt ctg ccg caa      834
Ser Val Glu Asp Phe Asn Ala Thr Leu Pro Lys Ser Arg Leu Pro Gln
    235                 240                 245

aac atc acc tta acc gaa gtg taa ttctctcttg ccgggcatct gcccggctat     888
Asn Ile Thr Leu Thr Glu Val
250                 255

ttcctctcag attctcctga tttgcataac cctgtttcag caagcttcgt catcataggc    948

tgctgttgaa gcttgcggcc gcactcgagc accaccacca ccaccactga gatccggctg   1008

ctaacaaagc ccgaaaggaa gctgagttgg ctgctgccac cgctgagcaa taactagc     1066
```

<210> 4
<211> 256
<212> PRT
<213> Escherichia coli K12 nfnB in pET-28(a)(+); pMKS2

<220>
<221> misc_feature
<222> (250)..(267)
<223> Cys tags

<220>
<221> misc_feature
<222> (160)..(177)
<223> His tags

<220>
<221> misc_feature
<222> (268)..(285)
<223> primer

<220>
<221> misc_feature
<222> (996)..(1010)
<223> primer

<400> 4

```
Met Gly Ser Ser His His His His His His Ser Ser Gly Leu Val Pro
1               5                   10                  15

Arg Gly Ser His Met Ala Ser Met Thr Gly Gly Gln Gln Met Gly Arg
            20                  25                  30

Gly Ser Cys Cys Cys Cys Cys Cys Asp Ile Ile Ser Val Ala Leu Lys
        35                  40                  45

Arg His Ser Thr Lys Ala Phe Asp Ala Ser Lys Lys Leu Thr Pro Glu
    50                  55                  60

Gln Ala Glu Gln Ile Lys Thr Leu Leu Gln Tyr Ser Pro Ser Ser Thr
65                  70                  75                  80
```

```
Asn Ser Gln Pro Trp His Phe Ile Val Ala Ser Thr Glu Glu Gly Lys
                85                  90                  95

Ala Arg Val Ala Lys Ser Ala Ala Gly Asn Tyr Val Phe Asn Glu Arg
            100                 105                 110

Lys Met Leu Asp Ala Ser His Val Val Val Phe Cys Ala Lys Thr Ala
        115                 120                 125

Met Asp Asp Val Trp Leu Lys Leu Val Val Asp Gln Glu Asp Ala Asp
    130                 135                 140

Gly Arg Phe Ala Thr Pro Glu Ala Lys Ala Ala Asn Asp Lys Gly Arg
145                 150                 155                 160

Lys Phe Phe Ala Asp Met His Arg Lys Asp Leu His Asp Asp Ala Glu
                165                 170                 175

Trp Met Ala Lys Gln Val Tyr Leu Asn Val Gly Asn Phe Leu Leu Gly
            180                 185                 190

Val Ala Ala Leu Gly Leu Asp Ala Val Pro Ile Glu Gly Phe Asp Ala
        195                 200                 205

Ala Ile Leu Asp Ala Glu Phe Gly Leu Lys Glu Lys Gly Tyr Thr Ser
    210                 215                 220

Leu Val Val Val Pro Val Gly His His Ser Val Glu Asp Phe Asn Ala
225                 230                 235                 240

Thr Leu Pro Lys Ser Arg Leu Pro Gln Asn Ile Thr Leu Thr Glu Val
                245                 250                 255
```

<210> 5
<211> 1221
<212> DNA
<213> Pseudomonas putida JLR11 prnB in pET-28(a)(+) ; pKMS6

<220>
<221> CDS
<222> (88)..(1029)
<223>

<220>
<221> misc_feature
<222> (190)..(225)
<223> primer

<220>

<221> misc_feature
<222> (190)..(207)
<223> cys tag

<220>
<221> misc_feature
<222> (936)..(956)
<223> primer

<400> 5

```
taatacgact cactataggg gaattgtgag cggataacaa ttcccctcta gaaataattt      60

tgtttaactt taagaaggag atatacc atg ggc agc agc cat cat cat cat cat     114
                              Met Gly Ser Ser His His His His His
                              1               5

cac agc agc ggc ctg gtg ccg cgc ggc agc cat atg gct agc atg act       162
His Ser Ser Gly Leu Val Pro Arg Gly Ser His Met Ala Ser Met Thr
10                  15                  20                  25

ggt gga cag caa atg ggt cgc gga tcc tgt tgc tgt tgc tgt tgc agc       210
Gly Gly Gln Gln Met Gly Arg Gly Ser Cys Cys Cys Cys Cys Cys Ser
                30                  35                  40

ctt caa gac gaa gca ctc aaa gcc tgg caa gcc cgt tat ggc gag cca       258
Leu Gln Asp Glu Ala Leu Lys Ala Trp Gln Ala Arg Tyr Gly Glu Pro
            45                  50                  55

gct aac tta cct gct gcc gac acc gtg atc gcg cag atg ttg cag cat       306
Ala Asn Leu Pro Ala Ala Asp Thr Val Ile Ala Gln Met Leu Gln His
```

```
        60                  65                  70

cga tca gta cgt gcc tac agc gat ctt cct gtg gat gag cag atg ctg       354
Arg Ser Val Arg Ala Tyr Ser Asp Leu Pro Val Asp Glu Gln Met Leu
    75                  80                  85

agc tgg gcg atc gcg gcg gcc cag tca gcc tcg act tcc tcg aac ctg       402
Ser Trp Ala Ile Ala Ala Ala Gln Ser Ala Ser Thr Ser Ser Asn Leu
90                  95                  100                 105

caa gct tgg agc gtg ctc gcc gtg cgg gat cgc gag cgt ctc gcg agg       450
Gln Ala Trp Ser Val Leu Ala Val Arg Asp Arg Glu Arg Leu Ala Arg
                110                 115                 120

ctt gcc cga ctg tcc ggt aac cag cgc cat gtc gag cag gca ccg ctg       498
Leu Ala Arg Leu Ser Gly Asn Gln Arg His Val Glu Gln Ala Pro Leu
            125                 130                 135

ttc ctg gtc tgg ctc gtg gac tgg tca cgc cta cgc cga cta gcc aga       546
Phe Leu Val Trp Leu Val Asp Trp Ser Arg Leu Arg Arg Leu Ala Arg
        140                 145                 150

acc ctt cag gca ccg act gca ggt atc gac tat tta gaa agc tac acc       594
Thr Leu Gln Ala Pro Thr Ala Gly Ile Asp Tyr Leu Glu Ser Tyr Thr
    155                 160                 165

gtc ggt gtt gta gat gca gct ctg gcc gct cag aac gcc gca cta gct       642
Val Gly Val Val Asp Ala Ala Leu Ala Ala Gln Asn Ala Ala Leu Ala
170                 175                 180                 185

ttc gag gcc caa gga ctg gga atc gtt tac atc ggc gga atg cgc aac       690
Phe Glu Ala Gln Gly Leu Gly Ile Val Tyr Ile Gly Gly Met Arg Asn
                190                 195                 200

cac ccg gaa gcg atg tcc gag gag ctt ggc ctg cca aac gac act ttc       738
His Pro Glu Ala Met Ser Glu Glu Leu Gly Leu Pro Asn Asp Thr Phe
            205                 210                 215

gct gta ttt ggc atg tgc gtc ggt cat ccc gat ccg gca cag ccc gcc       786
Ala Val Phe Gly Met Cys Val Gly His Pro Asp Pro Ala Gln Pro Ala
        220                 225                 230

gag atc aag cca cgc ctg gcg caa tca gtg gtg ctt cac cgt gag cgc       834
Glu Ile Lys Pro Arg Leu Ala Gln Ser Val Val Leu His Arg Glu Arg
    235                 240                 245

tat gag gcc acc gag gca gag gcg gtt tca gtt gct gcc tat gac cga       882
Tyr Glu Ala Thr Glu Ala Glu Ala Val Ser Val Ala Ala Tyr Asp Arg
250                 255                 260                 265

agg atg agc gac ttc caa cat cgt caa caa cgc gaa aac cgt tcc tgg       930
Arg Met Ser Asp Phe Gln His Arg Gln Gln Arg Glu Asn Arg Ser Trp
                270                 275                 280

tcc agc cag gcc gtg gaa cgt gta aaa gga gcg gat tca ctg agc gga       978
Ser Ser Gln Ala Val Glu Arg Val Lys Gly Ala Asp Ser Leu Ser Gly
            285                 290                 295

aga cac cgc ttg cga gat gca tta aac acc cta ggt ttc ggc ctg cgc      1026
```

```
Arg His Arg Leu Arg Asp Ala Leu Asn Thr Leu Gly Phe Gly Leu Arg
        300                 305                 310

tga gatagtgaga tatcccatgc ctattcccgc cgccctgaac cggagcacta          1079

atacctggca actttgcttg agctccgtcg acaagcttgc ggccgcactc gagcaccacc   1139

accaccacca ctgagatccg gctgctaaca aagcccgaaa ggaagctgag ttggctgctg   1199

ccaccgctga gcaataacta gc                                            1221
```

<210> 6
<211> 313
<212> PRT
<213> Pseudomonas putida JLR11 prnB in pET-28(a)(+) ; pKMS6

<220>
<221> misc_feature
<222> (190)..(225)
<223> primer
<220>
<221> misc_feature
<222> (190)..(207)
<223> cys tag
<220>
<221> misc_feature
<222> (936)..(956)
<223> primer

<400> 6

```
Met Gly Ser Ser His His His His His His Ser Ser Gly Leu Val Pro
1               5                   10                  15

Arg Gly Ser His Met Ala Ser Met Thr Gly Gly Gln Gln Met Gly Arg
            20                  25                  30

Gly Ser Cys Cys Cys Cys Cys Cys Ser Leu Gln Asp Glu Ala Leu Lys
        35                  40                  45
```

```
Ala Trp Gln Ala Arg Tyr Gly Glu Pro Ala Asn Leu Pro Ala Ala Asp
    50                  55                  60

Thr Val Ile Ala Gln Met Leu Gln His Arg Ser Val Arg Ala Tyr Ser
65                  70                  75                  80

Asp Leu Pro Val Asp Glu Gln Met Leu Ser Trp Ala Ile Ala Ala Ala
                85                  90                  95

Gln Ser Ala Ser Thr Ser Ser Asn Leu Gln Ala Trp Ser Val Leu Ala
            100                 105                 110

Val Arg Asp Arg Glu Arg Leu Ala Arg Leu Ala Arg Leu Ser Gly Asn
        115                 120                 125

Gln Arg His Val Glu Gln Ala Pro Leu Phe Leu Val Trp Leu Val Asp
    130                 135                 140

Trp Ser Arg Leu Arg Arg Leu Ala Arg Thr Leu Gln Ala Pro Thr Ala
145                 150                 155                 160

Gly Ile Asp Tyr Leu Glu Ser Tyr Thr Val Gly Val Val Asp Ala Ala
                165                 170                 175

Leu Ala Ala Gln Asn Ala Ala Leu Ala Phe Glu Ala Gln Gly Leu Gly
            180                 185                 190

Ile Val Tyr Ile Gly Gly Met Arg Asn His Pro Glu Ala Met Ser Glu
        195                 200                 205

Glu Leu Gly Leu Pro Asn Asp Thr Phe Ala Val Phe Gly Met Cys Val
    210                 215                 220

Gly His Pro Asp Pro Ala Gln Pro Ala Glu Ile Lys Pro Arg Leu Ala
225                 230                 235                 240

Gln Ser Val Val Leu His Arg Glu Arg Tyr Glu Ala Thr Glu Ala Glu
                245                 250                 255

Ala Val Ser Val Ala Ala Tyr Asp Arg Arg Met Ser Asp Phe Gln His
            260                 265                 270

Arg Gln Gln Arg Glu Asn Arg Ser Trp Ser Ser Gln Ala Val Glu Arg
        275                 280                 285
```

```
Val Lys Gly Ala Asp Ser Leu Ser Gly Arg His Arg Leu Arg Asp Ala
    290                 295                 300

Leu Asn Thr Leu Gly Phe Gly Leu Arg
305                 310
```

<210> 7
<211> 24
<212> DNA
<213> Escherichia coli

<400> 7
ggatccgata tcatttctgt cgcc 24

<210> 8
<211> 27
<212> DNA
<213> Escherichia coli

<400> 8
cgtcatcata ggctgctgtt gaagctt 27

<210> 9
<211> 42
<212> DNA
<213> Artificial Sequence

<220>
<223> Primer consisting of nfnB gene primer shown in SEQ ID4 with an ad ditional 6 cysteine codons

<400> 9
ggatcctgtt gctgttgctg ttgcgatatc atttctgtcg cc 42